(19) Europäisches Patentamt
European Patent Office
Office européen des brevets

(11) Veröffentlichungsnummer : **0 126 254**
**B1**

(12) **EUROPÄISCHE PATENTSCHRIFT**

(45) Veröffentlichungstag der Patentschrift : **11.11.87**

(21) Anmeldenummer : **84103804.5**

(22) Anmeldetag : **05.04.84**

(51) Int. Cl.⁴ : **C 07 D239/42**, C 07 D239/46, C 07 D239/52, C 07 D239/56, C 07 D239/58, A 01 N 43/54, A 61 K 31/505

# (54) N-(2-Nitrophenyl)-4-aminopyrimidin-Derivate als Mikrobizide.

(30) Priorität : **08.04.83 CH 1899/83**

(43) Veröffentlichungstag der Anmeldung : **28.11.84 Patentblatt 84/48**

(45) Bekanntmachung des Hinweises auf die Patenterteilung : **11.11.87 Patentblatt 87/46**

(84) Benannte Vertragsstaaten :
**AT BE CH DE FR IT LI LU NL SE**

(56) Entgegenhaltungen :
**EP-A- 0 073 328**
**GB-A- 1 388 825**
**US-A- 3 906 098**
**JOURNAL OF MIDICINAL AND PHARMACEUTICAL CHEMISTRY, Band 5, November 1962, Seiten 1085-1095, Washington, US; D.E. O'BRIEN et al.: "Pyrimidines. IX. 4- and 5-(substituted-anilino)pyrimidines"**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Hubele, Adolf, Dr.**
**Obere Egg 9**
**CH-4312 Magden (CH)**
Erfinder : **Eckhardt, Wolfgang, Dr.**
**Breslauerstrasse 14**
**D-7850 Lörrach (DE)**

(74) Vertreter : **Zumstein, Fritz, Dr. et al**
**Bräuhausstrasse 4**
**D-8000 München 2 (DE)**

**EP 0 126 254 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue N(2-Nitrophenyl)-4-amino-pyrimidin-Derivate der nachstehenden Formel I.

Die Erfindung betrifft ferner die Herstellung dieser Substanzen sowie mikrobizide Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von schädlichen Mikroorganismen, vorzugsweise pflanzenschädigenden Pilzen.

Bei den erfindungsgemässen Verbindungen handelt es sich um solche der allgemeinen Formel I :

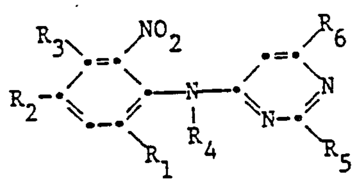

(I)

worin

$R_1$ für $NO_2$ oder $CF_3$ steht ;

$R_2$ für $NO_2$ oder $CF_3$ steht ;

$R_3$ Wasserstoff oder Halogen bedeutet ;

$R_4$ für Wasserstoff oder die Gruppe —C(O)$R_7$ steht, wobei $R_7$ unsubstituiertes oder durch Halogen, $C_1$-$C_3$-Alkoxy oder $C_1$-$C_3$-Alkylthio substituiertes $C_1$-$C_4$-Alkyl bedeutet ;

$R_5$ und $R_6$ unabhängig voneinander für Halogen, $C_1$-$C_6$-Alkoxy, durch Halogen oder $C_1$-$C_3$-Alkoxy substituiertes $C_1$-$C_6$-Alkyl, durch Halogen oder $C_1$-$C_3$-Alkoxy substituiertes $C_1$-$C_6$-Alkoxy, $C_1$-$C_6$-Alkylthio, durch Halogen oder $C_1$-$C_3$-Alkoxy substituiertes $C_1$-$C_6$-Alkylthio, $C_3$-$C_6$-Cycloalkoxy, $C_3$-$C_6$-Cycloalkylthio, ($C_2$-$C_5$-Alkenyl)-methoxy, ($C_2$-$C_5$-Alkenyl)-methylthio, ($C_2$-$C_5$-Alkinyl)-methoxy, $C_1$-$C_6$-Alkylsulfoxyl, $C_1$-$C_6$-Alkylsulfonyl, $C_1$-$C_6$-Sulfonyloxy, $C_3$-$C_6$-Alkenylsulfoxyl, $C_3$-$C_6$-Alkenylsulfonyl, $C_3$-$C_6$-Cycloalkylsulfoxyl oder für $C_3$-$C_6$-Cycloalkylsulfonyl stehen.

Unter dem Begriff Alkyl selbst oder als Bestandteil eines anderen Substituenten sind je nach Zahl der angegebenen Kohlenstoffatome beispielsweise folgende Gruppen zu verstehen : Methyl, Ethyl, Propyl, Butyl, Pentyl, Hexyl, usw., sowie ihre Isomeren, wie z. B. Isopropyl, Isobutyl, tert.-Butyl, Isopentyl usw. Alkenylmethyl steht z. B. für Allyl, Butenyl-(2), Butenyl-(3) usw., sowie Ketten mit mehreren Doppelbindungen. Alkinylmethyl bedeutet z. B. Propargyl, Butinyl-(2) usw., bevorzugt Propargyl. Durch Halogen substituiertes Alkyl steht insbesondere für ein- bis perhalogenierten Alkylsubstituenten, wie z. B. $CHCl_2$, $CH_2Cl$, $CCl_3$, $CF_3$, $CH_2CH_2Cl$, usw. Unter Halogen soll hier und im folgenden Fluor, Chlor, Brom oder Jod, vorzugsweise Cl, Br oder F verstanden werden. Cycloalkyl steht z. B. für Cyclopropyl, Cyclobutyl, Cyclopentyl, Cyclohexyl oder Cycloheptyl, bevorzugt für Cyclopropyl und Cyclohexyl.

Die Verbindungen der Formel I sind unter Normalbedingungen stabile Oele, Harze oder überwiegend kristalline Feststoffe, die sich durch äusserst wertvolle mikrobizide Eigenschaften auszeichnen. Sie lassen sich beispielsweise auf dem Agrarsektor oder verwandten Gebieten präventiv und kurativ zur Bekämpfung pflanzenschädigender Mikroorganismen einsetzen. Die erfindungsgemässen Wirkstoffe der Formel I zeichnen sich in weiten Anwendungskonzentrationen durch eine hohe fungizide Aktivität und problemlose Anwendung aus.

Auf Grund ihrer ausgeprägten mikrobiziden, insbesondere pflanzenfungiziden Wirkung sind folgende Untergruppen Ia bis Ii bevorzugt :

Gruppe Ia : Verbindungen der Formel I, worin $R_1$ für $NO_2$, $R_2$ für $CF_3$, $R_3$ für Chlor und $R_4$ für Wasserstoff stehen, $R_5$ Halogen, $C_1$-$C_6$-Alkoxy, durch Chlor oder Brom substituiertes $C_1$-$C_3$-Alkyl, durch $C_1$-$C_3$-Alkoxy substituiertes $C_1$-$C_3$-Alkoxy, $C_1$-$C_6$-Alkylthio, Cyclopentyloxy, Cyclohexyloxy, Cyclopentylthio, Cyclohexylthio, ($C_2$-$C_5$-Alkenyl)-methoxy, ($C_2$-$C_5$-Alkenyl)-methylthio, ($C_2$-$C_5$-Alkinyl)-methoxy, $C_1$-$C_3$-Alkylsulfoxyl, $C_1$-$C_6$-Alkylsulfonyl, $C_1$-$C_3$-Alkylsulfonyloxy oder Cyclohexylsulfonyl bedeutet ; und $R_6$ für Halogen steht.

Gruppe Ib : Verbindungen der Formel I, worin $R_1$ für $NO_2$, $R_2$ für $CF_3$, $R_3$ für Chlor oder Brom stehen, $R_5$ Halogen, $C_1$-$C_4$-Alkoxy, durch Chlor oder Brom substituiertes $C_1$-$C_3$-Alkyl, durch Methoxy substituiertes $C_1$-$C_2$ Alkoxy, $C_1$-$C_4$-Alkylthio, ($C_2$-$C_3$-Alkenyl)-methoxy, ($C_2$-$C_3$-Alkinyl)-methoxy oder $C_1$-$C_3$-Alkylsulfonyl bedeutet ; und $R_6$ für Fluor, Chlor oder Brom steht.

Innerhalb dieser Untergruppe Ib sind insbesondere jene Verbindungen der Formel I bevorzugt, worin $R_3$ und $R_6$ für Chlor stehen ; und $R_5$ Fluor, Chlor, Brom, $C_1$-$C_3$-Alkoxy, $C_1$-$C_3$-Alkylthio, $OCH_2CH_2OCH_3$, $OCH_2CH{=}CH_2$ oder $OCH_2C{\equiv}CH$ bedeutet. (= Gruppe Ib′).

Gruppe Ic : Verbindungen der Formel I, worin $R_1$ für $CF_3$, $R_2$ für $NO_2$, $R_3$ für Wasserstoff und $R_4$ für Wasserstoff stehen, $R_5$ Halogen, $C_1$-$C_6$-Alkoxy, durch Chlor oder Brom substituiertes $C_1$-$C_3$-Alkyl, durch

$C_1$-$C_3$-Alkoxy substituiertes $C_1$-$C_3$-Alkoxy, $C_1$-$C_6$-Alkylthio, Cyclopentyloxy, Cyclohexyloxy, Cyclopentylthio, Cyclohexylthio, ($C_2$-$C_5$-Alkenyl)-methoxy, ($C_2$-$C_5$-Alkenyl)-methylthio, ($C_2$-$C_5$-Alkinyl)-methoxy, $C_1$-$C_3$-Alkylsulfoxyl, $C_1$-$C_6$-Alkylsulfonyl, $C_1$-$C_3$-Alkylsulfonyloxy oder Cyclohexylsulfonyl bedeutet ; und $R_6$ für Halogen steht.

Gruppe Id : Verbindungen der Formel I, worin $R_1$ für $CF_3$, $R_2$ für $NO_2$, $R_3$ für Chlor oder Brom stehen $R_5$ Halogen, $C_1$-$C_4$-Alkoxy, durch Chlor oder Brom substituiertes $C_1$-$C_3$-Alkyl, durch Methoxy substituiertes $C_1$-$C_2$-Alkoxy, $C_1$-$C_4$-Alkylthio, ($C_2$-$C_3$-Alkenyl)-methoxy, ($C_2$-$C_3$-Alkinyl)-methoxy oder $C_1$-$C_3$-Alkylsulfonyl bedeutet ; und $R_6$ für Fluor, Chlor oder Brom steht.

Innerhalb dieser Untergruppe Id sind insbesondere jene Verbindungen der Formel I bevorzugt, worin $R_3$ und $R_6$ für Chlor stehen ; und $R_5$ Fluor, Chlor, Brom, $C_1$-$C_3$-Alkoxy, $C_1$-$C_3$-Alkylthio, $OCH_2CH_2OCH_3$, $OCH_2CH=CH_2$ oder $OCH_2C{\equiv}CH$ bedeutet. (= Gruppe Id').

Gruppe Ie : Verbindungen der Formel I, worin $R_1$ für $NO_2$, $R_2$ für $CF_3$, $R_3$ für Halogen und $R_4$ für Wasserstoff stehen, $R_5$ für $C_1$-$C_3$-Alkoxy, $C_1$-$C_3$-Alkylthio, $C_1$-$C_3$-Alkylsulfoxyl oder $C_1$-$C_3$-Alkylsulfonyl bedeutet und $R_6$ für Halogen steht.

Gruppe If : Verbindungen der Formel I, worin $R_1$ für $NO_2$, $R_2$ für $CF_3$, $R_3$ für Chlor und $R_4$ für Wasserstoff stehen, während $R_5$ und $R_6$ unabhängig voneinander halogen, $C_1$-$C_6$-Alkoxy, durch Chlor oder Brom substituiertes $C_1$-$C_3$-Alkyl, durch $C_1$-$C_3$-Alkoxy substituiertes $C_1$-$C_3$-Alkoxy, $C_1$-$C_6$-Alkylthio, Cyclopentyloxy, Cyclohexyloxy, Cyclopentylthio, Cyclohexylthio, ($C_2$-$C_5$-Alkenyl)-methoxy, ($C_2$-Alkenyl)-methylthio, ($C_2$-$C_5$-Alkinyl) methoxy, $C_1$-$C_3$-Alkylsulfoxyl, $C_1$-$C_6$-Alkylsulfonyl, $C_1$-$C_3$-Alkylsulfonyloxy oder Cyclohexylsulfonyl bedeuten.

Gruppe Ig : Verbindungen der Formel I, worin $R_1$ für $CF_3$, $R_2$ für $NO_2$, $R_3$ für Wasserstoff und $R_4$ für Wasserstoff stehen und $R_5$ und $R_6$ unabhängig voneinander $C_1$-$C_3$-Alkoxy, $C_1$-$C_3$-Alkylthio, $C_3$-Alkenyloxy, $C_1$-$C_3$-Alkoxy, $C_1$-$C_3$-Alkylthio, ($C_2$-Alkenyl)-methoxy, $C_1$-$C_3$-Alkenylsulfoxyl oder $C_1$-$C_3$-Alkenylsulfonyl bedeuten.

Gruppe Ih : Verbindungen der Formel I, worin $R_1$ für $CF_3$ oder $NO_2$ steht ; $R_2$ $CF_3$ oder $NO_2$ bedeutet ; $R_3$ Wasserstoff oder Chlor bedeutet ; $R_4$ für die Gruppe C(O)—$R_7$ steht, worin $R_7$ $C_1$-$C_3$-Alkyl, durch Halogen substituiertes $C_1$-$C_3$-Alkyl oder durch $C_1$-$C_3$-Alkoxy substituiertes $C_1$-$C_3$-Alkyl bedeutet ; $R_5$ für Halogen, $C_1$-$C_3$-Alkoxy oder $C_1$-$C_3$-Alkylthio steht und $R_6$ Chlor bedeutet.

Gruppe Ii : Verbindungen der Formel I, worin $R_1$ für $NO_2$, $R_2$ für $CF_3$, $R_3$ für Wasserstoff und $R_4$ für Wasserstoff stehen, $R_5$ Halogen, $C_1$-$C_6$-Alkoxy, durch Chlor oder Brom substituiertes $C_1$-$C_3$-Alkyl, durch $C_1$-$C_3$-Alkoxy substituiertes $C_1$-$C_3$-Alkoxy, $C_1$-$C_6$-Alkylthio, Cyclopentyloxy, Cyclohexyloxy, Cyclopentylthio, Cyclohexylthio, ($C_2$-$C_5$-Alkenyl)-methoxy, ($C_2$-$C_5$-Alkenyl)-methylthio, ($C_2$-$C_5$-Alkinyl)-methoxy, $C_1$-$C_3$-Alkylsulfoxyl, $C_1$-$C_6$-Alkylsulfonyl, $C_1$-$C_3$-Alkylsulfonyloxy oder Cyclohexylsulfonyl bedeutet ; und $R_6$ für Halogen steht.

Besonders bevorzugte Einzelsubstanzen sind z. B. :

N-(3'-Chlor-2',6'-dinitro-4'-trifluormethylphenyl)-4-amino-2,6-dichlor-pyrimidin ; (Nr. 1.1)
N-(2',4'-Dinitro-6'-trifluormethylphenyl)-4-amino-2,6-dichlorpyrimidin ; (Nr. 2.1)
N-(3'-Chlor-2',6'-dinitro-4'-trifluormethylphenyl)-4-amino-6-chlor-2-methylmercapto-pyrimidin ; (Nr. 1.5)
N-(2',6')-Dinitro-4'-trifluormethylphenyl)-4-amino-6-chlor-2-methylmercapto-pyrimidin ; (Nr. 7.32)
N-(3'-Chlor-2',6'-dinitro-4'-trifluormethylphenyl)-4-amino-6-chlor-2-methoxy-pyrimidin ; (Nr. 1.4)
N-(3'-Chlor-2',6'-dinitro-4'-trifluormethylphenyl)-4-amino-6-chlor-2-ethoxy-pyrimidin ; (Nr. 1.12)
N-(3'-Chlor-2',6'-dinitro-4'-trifluormethylphenyl)-4-amino-6-chlor-2-ethylmercapto-pyrimidin ; (Nr. 1.63)
N-(3'-Chlor-2',6'-dinitro-4'-trifluormethylphenyl)-4-amino-6-chlor-2-sek.butylmercapto-pyrimidin ; (Nr. 1.80)
N-(2',6'-Dinitro-4'-trifluormethylphenyl)-4-amino-2,6-dichlorpyrimidin ; (Nr. 7.1)
N-(2',4'-Dinitro-6'-trifluormethylphenyl)-4-amino-2-methoxy-6-chlorpyrimidin ; (Nr. 2.7)
N-(2',4'-Dinitro-6'-trifluormethylphenyl)-4-amino-2-ethoxy-6-chlorpyrimidin ; (Nr. 2.6).

Die Verbindungen der Formel I werden erfindungsgemäss dadurch hergestellt, dass man eine Verbindung der Formel II

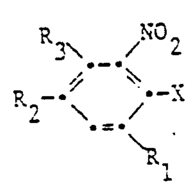

(II)

in Gegenwart einer Base mit einem Pyrimidin-Derivat der Formel III

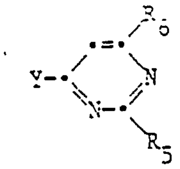 (III)

zu einer Verbindung der Formel I′

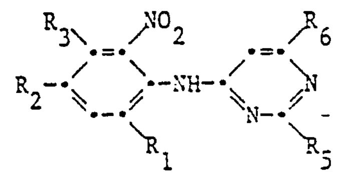 (I′)

umsetzt, und letztere zur Herstellung N-acylierter Derivate mit einem reaktionsfähigen Derivat der Carbonsäure IV

$R_7COOH$ (IV)

N-acyliert, wobei die Substituenten $R_1$ bis $R_7$ die unter Formel I angegebenen Bedeutungen haben und X und Y für $NH_2$ oder Halogen stehen, wobei in dem Fall, in dem X für Halogen steht, Y $NH_2$ bedeutet und in dem Fall in dem X für $NH_2$ steht, Y Halogen bedeutet.

Für die Herstellung der Verbindungen der Formel I bzw. I′ sind folgende Reaktionsbedingungen vorteilhaft :

Die N-Alkylierung von (II) mit (III) zu (I′), sowie die N-Acylierung von (I′) mit (IV) zu (I) erfolgen unter Halogenwasserstoffabspaltung. Die Reaktionstemperaturen liegen bei der N-Alkylierung zwischen — 20° und 150 °C, vorzugsweise — 20° und + 30 °C, bei der N-Acylierung zwischen 0° und + 180 °C, vorzugsweise 0° und 150 °C bzw. am Siedepunkt des Lösungsmittels bzw. Lösungsmittelgemisches. In beiden Fällen ist die Verwendung von säurebindenden Mitteln bzw. Kondensationsmitteln vorteilhaft. Als solche kommen organische und anorganische Basen in Betracht, z. B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide und Hydroxide, Carbonate und Hydrogencarbonate von Alkali- und Erdalkalimetallen sowie Alkaliacetate.

Die Reaktionen können in Anwesenheit von reaktionsinerten Lösungs- oder Verdünnungsmitteln durchgeführt werden. In Frage kommen beispielsweise aliphatische und aromatische Kohlenwasserstoffe wie Benzol, Toluol, Xylole, Petrolether ; halogenierte Kohlenwasserstoffe wie Chlorbenzol, Methylenchlorid, Ethylenchlorid, Chloroform, Tetrachlorkohlenstoff, Tetrachlorethylen ; Ether und etherartige Verbindungen wie Dialkylether (Diethylether, Diisopropylether, tert.-Butylmethylether usw.), Anisol, Dioxan, Tetrahydrofuran ; Nitrile wie Acetonitril, Propionitril ; N,N-dialkylierte Amide wie Dimethylformamid ; Dimethylsulfoxid ; Ketone wie Aceton, Diethylketon, Methylethylketon und Gemische solcher Lösungsmittel untereinander.

Die Reaktion (II) und (III) kann auch in einem wässrigen Zweiphasensystem nach dem allgemein bekannten Prinzip der Phasentransferkatalyse durchgeführt werden.

Für die organische, mit Wasser nicht mischbare Phase kommen dabei z. B. folgende Lösungsmittel in Frage : Aliphatische und aromatische Kohlenwasserstoffe, wie Pentan, Hexan, Cyclohexan, Petrolether, Ligroin, Benzol, Toluol, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw. oder aliphatische Ether, wie Diethylether, Diisopropylether, t-Butylmethylether usw.. Beispiele geeigneter Phasentransfer-Katalysatoren sind : Tetraalkylammoniumhalogenide, -hydrogensulfate oder -hydroxide wie Tetrabutylammoniumchlorid, -bromid, -jodid ; Triethylbenzylammoniumchlorid, -bromid ; Tetrapropylammoniumchlorid, -bromid, -jodid ; usw. Als Phasentransfer-Katalysatoren kommen auch Phosphonium-Salze in Betracht. Die Reaktionstemperaturen liegen im allgemeinen zwischen — 30° und 130 °C, bzw. am Siedepunkt des Lösungsmittels oder Lösungsmittelgemisches.

Im übrigen kann bei der Herstellung aller hierin genannten Ausgang-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether ; halogenierte Kohlenwasserstoffe wie Chlorbenzol, Methylenchlorid, Ethylenchlorid, Chloroform, Tetrachlorkohlenstoff, Tetrachlorethylen ; Ether und etherartige Verbindungen wie Dialkylether (Diethylether, Diisopropylether, tert.-Butylmethylether usw.), Anisol, Dioxan, Tetrahydrofuran ; Nitrile wie Acetonitril, Propionitril ; N,N-dialkylierte Amide wie Dimethylformamid ; Dimethylsulfoxid ; Ketone wie Aceton, Diethylketon, Methylethylketon und Gemische solcher Lösungsmittel untereinander. In manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon oder in gewissen Fällen auch Kohlendioxid.

Das beschriebene Herstellungsverfahren ist, einschliesslich aller Teilschritte, ein wichtiger Bestandteil der vorliegenden Erfindung.

In der britischen Patentschrift Nr 1 388 825 werden heterocyclische Verbindungen, darunter auch Pyrimidinderivate, als Schädlingsbekämpfungsmittel, z. B. auch zur Bekämpfung phytopathogener Mikroorganismen, beschrieben.

Es wurde überraschend festgestellt, dass Verbindungen der Formel I ein für praktische Bedürfnisse sehr günstiges Mikrobizid-Spektrum gegen phytopathogene Pilze und Bakterien aufweisen. Sie besitzen sehr vorteilhafte kurative, systemische und insbesondere präventive Eigenschaften und lassen sich zum Schutz von zahlreichen Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe der Formel I sind gegen die den folgenden Klassen angehörenden phytopathogenen Pilze wirksam : Fungi imperfecti (z. B. Botrytis, Helminthosporium, Fusarium Septoria, Cercospora und Alternaria) ; Basidiomyceten (z. B. die Gattungen Hemileia, Rhizocotonia, Puccinia) ; insbesondere wirken sie gegen die Klasse der Ascomyceten (z. B. Venturia, Podosphaera, Erysiphe, Monilinia, Uncinula). Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten : Getreide ; (Weizen, Gerste, Roggen, Hafer, Reis, Sorghum und Verwandte) ; Rüben : (Zucker- und Futterrüben) ; Kern-, Stein- und Beerenobst : (Aepfel, Birnen, Pflaumen, Pfirsiche, Mandeln, Kirschen, Erd-, Him- und Brombeeren) ; Hülsenfrüchte : (Bohnen, Linsen, Erbsen, Soja) ; Oelkulturen : (Raps, Senf, Mohn, Oliven, Sonnenblumen, Kokos, Rizinus, Kakao, Erdnüsse) ; Gurkengewächse : (Kürbis, Gurken, Melonen) ; Fasergewächse : (Baumwolle, Flachs, Hanf, Jute) ; Citrusfrüchte : (Orangen, Zitronen, Pampelmusen, Mandarinen) ; Gemüsesorten : (Spinat, Kopfsalat, Spargel, Kohlarten, Möhren, Zwiebeln, Tomaten, Kartoffeln, Paprika) ; Lorbeergewächse : (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z. B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z. B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z. B. die gezielte Behandlung der Pflanzenstengel oder der Knospen.

Die Verbindungen der Formel I werden dabei in unveränderter Form oder vorzugsweise zusammen

mit den in der Formulierungstechnik üblichen Hilfsmittel eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha ; bevorzugt 100 g bis 2 kg AS/ha, insbesondere bei 200 g bis 600 g AS/ha.

Die Formulierungen d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden. Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z. B. Phosphatidylethanolamin, Phosphatidylserin, Phosphatidylcholin, Sphingomyelin, Phosphatidylinosit, Phosphatidylglycerin, Lysolecithin, Plasmalogene oder Cardiolipin, die man beispielsweise aus tierischen oder pflanzlichen Zellen, insbesondere aus Hirn, Herz, Leber, Eidotter oder Sojabohnen gewinnen kann. Verwendbare Handelsmischungen sind z. B. Phosphatidylcholin-Mischungen. Synthetische Phospholipide sind z. B. Dioctanoylphosphatidylcholin und Dipalmitoylphosphatidylcholin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z. B. die Na$^{-}$, Ca$^{-}$ oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 g Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1

bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z. B. das Stearyltrimethylammoniumchlorid oder das Benzyldi(2-chlorethyl) ethylammoniumbromid.

Die in der Formulierungstechnik gebräuchlichen Tenside sind u. a. in folgenden Publikationen beschrieben :

« Mc Cutcheon's Detergents and Emulsifiers Annual » BC Publishing Corp., Ringwood New Jersey, 1980 Sisely and Wood, « Encyclopedia of Surgace Active Agents », Chemical Publishing Co., Inc. New York, 1980.

Die agrochemischen Zubereitungen enthalten in der Regel 0,1 bis 99 %, insbesondere 0,1 bis 95 % Wirkstoff der Formel I, 99,9 bis 1 %, insbesondere 99,8 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbaucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht.

## Herstellungsbeispiele

### Beispiel H1 : Herstellung von

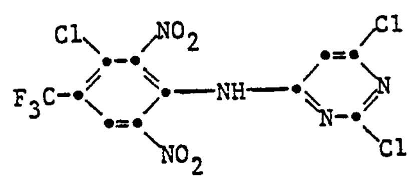

(1.1)

N-(3'Chlor-2',6'-dinitro-4'-trifluormethylphenyl)-4-amino-2,6-dichlorpyrimidin

Eine Lösung von 19,1 Teilen 2,6-Dichlor-4-amino-pyrimidin in 430 ml Tetrahydrofuran wird bei Raumtemperatur unter Rühren portionsweise mit 16,5 Teilen 85 %igem, pulverisiertem Kaliumhydroxid versetzt, wobei die Temperatur innerhalb einer halben Stunde auf ca. 24 °C ansteigt. Das Reaktionsgemisch wird auf 0 °C abgekühlt, und zu der beigen Suspension werden innerhalb von 1 Stunde 35,4 Teile 2,4-Dichlor-3,5-dinitro-benzotrifluorid in 120 ml Tetrahydrofuran zugetropft, wobei sich die Suspension rot verfärbt. Nach 14 stündigem Rühren bei Raumtemperatur wird das Reaktionsgemisch auf Eiswasser gegossen, mit 15 ml konzentrierter Salzsäure angesäuert und zweimal mit je 300 ml Essigsäureethylester extrahiert. Die vereinigten Extrakte werden zweimal mit je 100 ml Wasser gewaschen, über Natriumsulfat getrocknet, filtriert und eingedampft. Der ölige Rückstand wird durch Zusatz von Petrolether zur Kristallisation gebracht und nach dem Abfiltrieren aus Chloroform umkristallisiert. Man erhält beige Kristalle mit Smp. 174-177 °C.

### Beispiel H2 : Herstellung von

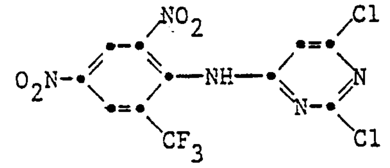

(2.1)

N-(2',4'-Dinitro-6'-trifluormethylphenyl)-4-amino-2,6-dichlorpyrimidin

Zu einer Lösung von 8,2 Teilen 2,6-Dichlor-4-amino-pyrimidin in 200 ml absolutem Tetrahydrofuran

werden 7,24 Teile 85 %iges, pulverisiertes Kaliumhydroxid gegeben. Das Gemisch wird auf 0 °C abgekühlt und unter Rühren portionsweise innerhalb von einer Stunde mit 13,5 Teilen 2-Chlor-3,5-dinitrobenzotrifluorid in 100 ml absolutem Tetrahydrofuran versetzt ; dabei schlägt die Farbe der Reaktionslösung von gelb nach rot um. Nach 12 stündigem Rühren wird die Reaktionslösung auf 500 ml Eiswasser gegossen, mit 8 ml konzentrierter Salzsäure angesäuert und zweimal mit je 200 ml Essigsäureethylester extrahiert. Die vereinigten Extrakte werden mit 50 ml Wasser gewaschen, über Natriumsulfat getrocknet, filtriert und eingedampft. Das Rohprodukt wird aus Methanol umkristallisiert. Smp. 198-200 °C.

Beispiel H3 : Herstellung von

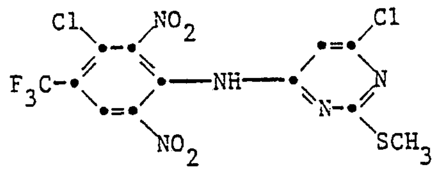

(1.5)

N-(3'-Chlor-2',6'-dinitro-4'-trifluormethylphenyl)-4-amino-6-chlor-2-methylmercapto-pyrimidin

7,9 Teile fein pulverisiertes, 85 %iges Kaliumhydroxid werden in 70 ml Dimethylsulfoxid gelöst. Dazu wird eine Lösung von 17,6 Teilen 4-Amino-6-chlor-2-methylmercaptopyrimidin in 80 ml Dimethylsulfoxid bei ca. 15 °C zugetropft und das Reaktionsgemisch eine halbe Stunde bei Raumtemperatur gerührt. Anschliessend werden bei ca. 115 °C 30,5 Teile 1,3-Dichlor-2,6-dinitro-4-trifluormethylbenzol in 100 ml Dimethylsulfoxid zugetropft, das Reaktionsgemisch über Nacht bei Raumtemperatur gerührt, dann auf 2 Liter Eiswasser gegossen und mit Essigsäureethylester extrahiert. Die vereinigten Extrakte werden mehrmals mit Wasser gewaschen, über Natriumsulfat getrocknet, filtriert und eingeengt. Das Rohprodukt wird säulenchromatographisch (Kieselgel/Dichlormethan) gereinigt. Ausbeute 22,3 Teile, Smp. 171-175 °C.

Auf analoge Weise werden auch die nachfolgend aufgeführten Substanzen der Formel I hergestellt :

(Siehe Tabellen Seite 9 ff.)

Tabelle 1 : Verbindungen der Formel

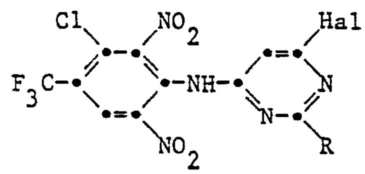

| Verb. Nr. | R | Hal | Physik. Konst. [°C] |
|---|---|---|---|
| 1.1 | Cl | Cl | Smp. 174-177 |
| 1.2 | $OCH_3$ | F | Harz |
| 1.3 | Br | Br | Harz |
| 1.4 | $OCH_3$ | Cl | Smp. 199-201 |
| 1.5 | $SCH_3$ | Cl | Smp. 171-175 |
| 1.6 | $O-C(CH_3)(C_2H_5)-C\equiv CH$ | Cl | |
| 1.7 | $OC_2H_5$ | F | Harz |
| 1.8 | $OCH_2CH_2OCH_3$ | Cl | Smp. 128-130 |
| 1.9 | F | F | Harz |
| 1.10 | $OC_3H_7$-i | F | |
| 1.11 | $OCH_2CH_2=CHCH_3$ | Cl | |
| 1.12 | $OC_2H_5$ | Cl | Smp. 164-169 |
| 1.13 | $OCH_2CH(C_2H_5)_2$ | F | |
| 1.14 | $OCH_2CH=CH_2$ | Cl | semikristallin |
| 1.15 | $OC_3H_7$-n | Cl | Smp. 165-166 |
| 1.16 | $OCH_2CH_2OC_2H_5$ | Cl | |

Tabelle 1 (Fortsetzung)

| Verb. Nr. | R | Hal | Physik. Konst. [°C] |
|---|---|---|---|
| 1.17 | J | J | |
| 1.18 | $SCH_3$ | F | |
| 1.19 | $OC_3H_7$-i | Cl | |
| 1.20 | $OCH_2C{\equiv}CH$ | Cl | semikristallin |
| 1.21 | $SC_2H_5$ | F | |
| 1.22 | $OC_4H_9$-n | Cl | |
| 1.23 | $SCH_2CH{=}CH_2$ | Cl | |
| 1.24 | $OC(CH_3)_2C{\equiv}CH$ | Cl | |
| 1.25 | $SC_3H_7$-i | F | |
| 1.26 | $O(CH_2)_6Cl$ | Cl | |
| 1.27 | $OCH(CH_3)C_2H_5$ | Cl | semikristallin |
| 1.28 | $OCH(CH_3)C{\equiv}CH$ | Cl | |
| 1.29 | O-Cyclohexyl | Cl | |
| 1.30 | O-$C_4H_9$-t | Cl | |
| 1.31 | S-$C_4H_9$-t | F | |
| 1.32 | $O(CH_2)_3Cl$ | Cl | Harz |
| 1.33 | $S(O)C_2H_5$ | Cl | |
| 1.34 | $OCH_2CH_2Cl$ | F | |
| 1.35 | S-Cyclohexyl | Cl | |
| 1.36 | $S(O)CH_3$ | Cl | |
| 1.37 | $OCH_2CF_3$ | Cl | semikristallin |
| 1.38 | $OCH_2CH_2OCH_3$ | F | |

Tabelle 1 (Fortsetzung)

| Verb. Nr. | R | Hal | Physik. Konst. [°C] |
|---|---|---|---|
| 1.39 | $OC_5H_{11}$-n | Cl | |
| 1.40 | $S(O)_2C_2H_5$ | Cl | |
| 1.41 | $OCH_2CCl_3$ | Cl | |
| 1.42 | $S(O)_2CH_3$ | Cl | Zers. ab 102 |
| 1.43 | $OCH_2CH=CH_2$ | F | |
| 1.44 | $OCH_2-CH(CH_3)C_2H_5$ | Cl | |
| 1.45 | $OCH_2CH(Br)CH_2Br$ | Cl | |
| 1.46 | $OC(CH_3)_2C_2H_5$ | Cl | |
| 1.47 | $S(O)_2C_3H_7$-n | Cl | |
| 1.48 | $OCH(CH_3)CH_2CH_2CH_3$ | Cl | |
| 1.49 | $OCH_3$ | Br | Harz |
| 1.50 | $OC_2H_5$ | J | |
| 1.51 | $OCH_2-C\equiv CH$ | F | |
| 1.52 | $OC_6H_{13}$-n | Cl | |
| 1.53 | $OCH_2CH_2Br$ | Cl | semikristallin |
| 1.54 | $OCH_2CH(C_2H_5)_2$ | Cl | |
| 1.55 | $SCH_3$ | Br | |
| 1.56 | $S(O)_2CH_3$ | F | |
| 1.57 | $OC_2H_5$ | Br | Harz |
| 1.58 | $OCH_2CH(CH_3)CH_2CH_2CH_3$ | Cl | |
| 1.59 | $S(O)_2CH_3$ | Br | |
| 1.60 | $OCH_3$ | J | |

Tabelle 1 (Fortsetzung)

| Verb. Nr. | R | Hal | Physik. Konst. [°C] |
|---|---|---|---|
| 1.61 | $S(O)_2C_2H_5$ | F | |
| 1.62 | $OC(CH_3)(C_2H_5)C_2H_5$ | Cl | |
| 1.63 | $SC_2H_5$ | Cl | Smp. 175-176 |
| 1.64 | $SC_5H_{11}$-n | Cl | |
| 1.65 | $S(O)_2C_4H_9$-n | Cl | |
| 1.66 | $OCH(CH_3)CH_2CH(CH_3)_2$ | Cl | |
| 1.67 | $S(O)_3C_3H_7$-i | F | |
| 1.68 | $SC_3H_7$-n | Cl | |
| 1.69 | $SC_6H_{13}$-n | Cl | |
| 1.70 | $OCH_2CH_2Cl$ | Cl | |
| 1.71 | $SCH_3$ | J | |
| 1.72 | $S(O)_2CH_3$ | J | |
| 1.73 | $SC_3H_7$-i | Cl | |
| 1.74 | $SC_2H_5$ | Br | Harz |
| 1.75 | $S(O)_2C_6H_{13}$-n | Cl | |
| 1.76 | $OC_3H_7$-i | J | |
| 1.77 | $SC_4H_9$-n | Cl | |
| 1.78 | $SC_3H_7$-n | J | |
| 1.79 | $S(O)_2C_2H_5$ | Br | |
| 1.80 | $SCH(CH_3)C_2H_5$ | Cl | Smp. 152-155 |
| 1.81 | $OCH_2C{\equiv}CH$ | J | |

Tabelle 1 (Fortsetzung)

| Verb. Nr. | R | Hal | Physik. Konst. [°C] |
|---|---|---|---|
| 1.82 | $SC_4H_9$-t | Cl | |
| 1.83 | $S(O)_2$Cyclohexyl | Cl | |
| 1.84 | $OCH_2CH(CH_3)_2$ | Cl | Smp. 174-177 |

Tabelle 2 : Verbindung der Formel

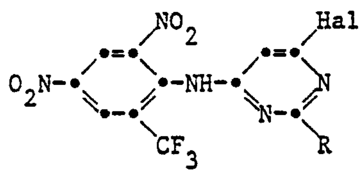

| Verb. Nr. | R | Hal | Physik. Konst. [°C] |
|---|---|---|---|
| 2.1 | Cl | Cl | Smp. 198-200 |
| 2.2 | $OCH_3$ | F | Harz |
| 2.3 | $OC_3H_7$-i | Cl | Harz |
| 2.4 | $S(O)_2CH_3$ | Cl | |
| 2.5 | F | F | Harz |
| 2.6 | $OC_2H_5$ | Cl | Smp. 150-152 |
| 2.7 | $OCH_3$ | Cl | Smp. 170-172 |
| 2.8 | $OC_2H_5$ | F | Harz |
| 2.9 | $S(O)_2C_2H_5$ | Cl | |
| 2.10 | $OC_3H_7$-n | Cl | Smp. 76-78 |

Tabelle 2 (Fortsetzung)

| Verb. Nr. | R | Hal | Physik. Konst. [°C] |
|---|---|---|---|
| 2.11 | $S(O)CH_3$ | Cl | |
| 2.12 | Br | Br | Harz |
| 2.13 | $OC_4H_9$-n | Cl | |
| 2.14 | S-Cyclohexyl | Cl | |
| 2.15 | J | J | |
| 2.16 | $OC_3H_7$-i | F | |
| 2.17 | $OC_4H_9$-t | Cl | |
| 2.18 | $S(O)C_2H_5$ | Cl | |
| 2.19 | $SCH_3$ | F | |
| 2.20 | $S(O)_2C_3H_7$-n | Cl | |
| 2.21 | $OCH_2CH(CH_3)C_2H_5$ | Cl | |
| 2.22 | $OCH(CH_3)C_2H_5$ | Cl | semikristallin |
| 2.23 | $OC_5H_{11}$-n | Cl | |
| 2.24 | O-Cyclohexyl | Cl | |
| 2.25 | $SC_2H_5$ | F | |
| 2.26 | $OC_6H_{13}$-n | Cl | |
| 2.27 | $OCH_3$ | Br | Harz |
| 2.28 | $OC_6H_{13}$-n | F | |
| 2.29 | $SC_4H_9$-t | F | |
| 2.30 | $OC_2H_5$ | Br | Harz |

Tabelle 2 (Fortsetzung)

| Verb. Nr. | R | Hal | Physik. Konst. [°C] |
|---|---|---|---|
| 2.31 | $OCH_2CH(CH_3)CH_2CH_2CH_3$ | Cl | |
| 2.32 | $SCH_3$ | Cl | Smp. 182-188 |
| 2.33 | $OC_3H_7$-i | Br | |
| 2.34 | $OCH_2CH(C_2H_5)_2$ | Cl | |
| 2.35 | $OCH_2CH_2OCH_3$ | F | |
| 2.36 | $SCH_3$ | Br | |
| 2.37 | $OC(CH_3)_2C{\equiv}CH$ | Cl | |
| 2.38 | $S(O)_2CH_3$ | F | |
| 2.39 | $SC_4H_9$-n | Cl | |
| 2.40 | $SC_2H_5$ | Cl | Smp. 90-93 |
| 2.41 | $SCH_2CH{=}CH_2$ | Cl | |
| 2.42 | $OCH_2CF_3$ | Cl | semikristallin |
| 2.43 | $S(O)_2C_2H_5$ | F | |
| 2.44 | $SC_2H_5$ | Br | Harz |
| 2.45 | $SC_3H_7$-n | Cl | |
| 2.46 | $SC_3H_7$-i | Br | |
| 2.47 | $SC_3H_7$-i | Cl | |
| 2.48 | $OC(CH_3)_2C{\equiv}CH$ | Cl | |
| 2.49 | $OCH_2C{\equiv}CH$ | Cl | semikristallin |
| 2.50 | $OCH_2CH_2Br$ | Br | |
| 2.51 | $S(O)_2CH_3$ | Br | |

Tabelle 2 (Fortsetzung)

| Verb. Nr. | R | Hal | Physik. Konst. [°C] |
|---|---|---|---|
| 2.52 | $S(O)_2C_6H_{13}$-n | F | |
| 2.53 | $SC_4H_9$-n | Cl | |
| 2.54 | $SO_2C_2H_5$ | Br | |
| 2.55 | $OCH_2CH=CH_2$ | Cl | semikristallin |
| 2.56 | $OCH_3$ | J | |
| 2.57 | $SC_4H_9$-t | Cl | |
| 2.58 | $OC_2H_5$ | J | |
| 2.59 | $SC_6H_{13}$-n | Cl | |
| 2.60 | $OCH_2CH_2OC_2H_5$ | Cl | |
| 2.61 | $OCH_2CH(CH_3)_2$ | Cl | Smp. 144-145 |
| 2.62 | $SCH_3$ | J | |
| 2.63 | $OCH_2CH_2Cl$ | Cl | |
| 2.64 | $S(O)_2CH_3$ | J | |
| 2.65 | $S(O)_2C_2H_5$ | J | |
| 2.66 | $O(CH_2)_3Cl$ | Cl | Harz |
| 2.67 | $SC_2H_5$ | J | |
| 2.68 | $OCH_2CH=CH_2$ | J | |
| 2.69 | $OCH_2CH_2OCH_3$ | Cl | Smp. 160-162 |
| 2.70 | $OCH_2C\equiv CH$ | J | |

Tabelle 3 : Verbindung der Formel

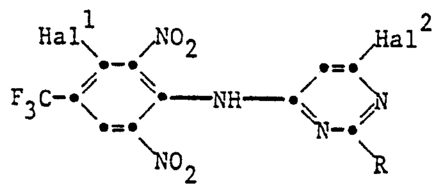

| Verb. Nr. | $Hal^1$ | $Hal^2$ | R | Physik. Konst. [°C] |
|---|---|---|---|---|
| 3.1 | Br | Cl | $OCH_3$ | |
| 3.2 | F | Br | $OCH_3$ | |
| 3.3 | F | J | $OCH_3$ | |
| 3.4 | F | Cl | $OCH_3$ | |
| 3.5 | Br | Cl | $SCH_3$ | |
| 3.6 | F | Br | $SCH_3$ | |
| 3.7 | F | Cl | $SCH_3$ | |
| 3.8 | F | Br | $S(O)_2CH_3$ | |
| 3.9 | Br | Cl | $S(O)_2CH_3$ | |
| 3.10 | Br | Cl | $SC_3H_7$-i | |
| 3.11 | F | Cl | $S(O)_2CH_3$ | |
| 3.12 | F | F | $SCH_3$ | |
| 3.13 | J | Cl | $OCH_3$ | |
| 3.14 | Br | Br | $OCH_3$ | |
| 3.15 | Br | F | $OCH_3$ | |
| 3.16 | J | Cl | $SCH_3$ | |
| 3.17 | Br | F | $SCH_3$ | |

Tabelle 3 (Fortsetzung)

| Verb. Nr. | $Hal^1$ | $Hal^2$ | R | Physik. Konst. [°C] |
|---|---|---|---|---|
| 3.18 | Br | Br | $SCH_3$ | |
| 3.19 | J | Cl | $S(O)_2CH_3$ | |
| 3.20 | J | Cl | $SC_2H_5$ | |
| 3.21 | Br | J | $OCH_3$ | |
| 3.22 | J | Cl | $OC_3H_7$ | |
| 3.23 | J | Cl | $OC_2H_5$ | |

Tabelle 4 : Verbindungen der Formel

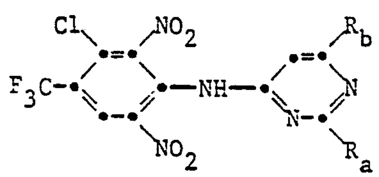

| Verb. Nr. | $R_a$ | $R_b$ | Physik. Konst. [°C] |
|---|---|---|---|
| 4.1 | $OCH_3$ | $SCH_3$ | |
| 4.2 | $SC_3H_7$-i | $OCH_3$ | |
| 4.3 | $S(O)_2CH_3$ | $OCH_3$ | |
| 4.4 | $OCH_3$ | $OCH_3$ | Smp. 136-140 |
| 4.5 | $SC_3H_7$-i | $OC_2H_5$ | |
| 4.6 | $S(O)_2CH_3$ | $OC_2H_5$ | |

Tabelle 4 (Fortsetzung)

| Verb. Nr. | $R_a$ | $R_b$ | Physik. Konst. [°C] |
|---|---|---|---|
| 4.7 | $OCH_3$ | $OC_2H_5$ | |
| 4.8 | $S(O)_2CH_3$ | $OC_3H_7$-i | |
| 4.9 | $OCH_3$ | $SC_2H_5$ | |
| 4.10 | $SC_3H_7$-i | $SCH_3$ | |
| 4.11 | $S(O)_2CH_3$ | $SCH_3$ | |
| 4.12 | $OCH_3$ | $SC_3H_7$-i | |
| 4.13 | $S(O)_2CH_3$ | $SC_2H_5$ | |
| 4.14 | $SC_3H_7$-i | $SC_2H_5$ | |
| 4.15 | $S(O)_2CH_3$ | $SC_4H_9$-t | |
| 4.16 | $OCH_3$ | $SC_4H_9$-t | |
| 4.17 | $S(O)_2CH_3$ | $S(O)_2CH_3$ | |
| 4.18 | $SC_3H_7$-i | $SC_3H_7$-i | |
| 4.19 | $S(O)_2CH_3$ | $OCH_2CH{=}CH_2$ | |
| 4.20 | $OCH_3$ | $OCH_2CH{=}CH_2$ | |
| 4.21 | $SO_2CH_3$ | $OCH_2CH{=}CHCH_3$ | |
| 4.22 | $SC_3H_7$-i | $SC_4H_9$-t | |
| 4.23 | $OCH_3$ | $OCH_2CH_2Cl$ | |
| 4.24 | $OCH_3$ | $S(O)_2CH_3$ | |
| 4.25 | $S(O)_2CH_3$ | $S(O)_2C_2H_5$ | |
| 4.26 | $SC_3H_7$-i | $OCH_2CH{=}CH_2$ | |

Tabelle 4 (Fortsetzung)

| Verb. Nr. | $R_a$ | $R_b$ | Physik. Konst. [°C] |
|---|---|---|---|
| 4.27 | $OCH_3$ | $S(O)_2C_2H_5$ | |
| 4.28 | $S(O)_2C_2H_5$ | $S(O)_2CH_3$ | |
| 4.29 | $S(O)_2C_2H_5$ | $OCH_3$ | |
| 4.30 | $OC_2H_5$ | $OCH_3$ | |
| 4.31 | $S(O)_2C_2H_5$ | $S(O)_2C_2H_5$ | |
| 4.32 | $SC_3H_7$-i | $OCH_2CH_2Cl$ | |
| 4.33 | $OC_2H_5$ | $OC_2H_5$ | |
| 4.34 | $SC_4H_9$-t | $OCH_3$ | |
| 4.35 | $OC_2H_5$ | $SC_2H_5$ | |
| 4.36 | $SC_4H_9$-t | $OC_2H_5$ | |
| 4.37 | $S(O)_2C_2H_5$ | $OC_2H_5$ | |
| 4.38 | $OC_2H_5$ | $SC_3H_7$-i | |
| 4.39 | $S(O)_2C_2H_5$ | $SCH_3$ | |
| 4.40 | $SC_4H_9$-t | $SCH_3$ | |
| 4.41 | $OC_2H_5$ | $SC_4H_9$-t | |
| 4.42 | $S(O)_2C_2H_5$ | $SC_2H_5$ | |
| 4.43 | $SC_4H_9$-t | $SC_2H_5$ | |
| 4.44 | $OC_2H_5$ | $OCH_2CH=CH_2$ | |
| 4.45 | $S(O)_2C_2H_5$ | $SC_3H_7$-i | |
| 4.46 | $OC_2H_5$ | $OCH_2CH_2Cl$ | |

Tabelle 4 (Fortsetzung)

| Verb. Nr. | $R_a$ | $R_b$ | Physik. Konst. [°C] |
|---|---|---|---|
| 4.47 | $OC_2H_5$ | $SCH_3$ | |
| 4.48 | $S(O)_2C_2H_5$ | $SC_4H_9$-t | |
| 4.49 | $OC_2H_5$ | $S(O)_2CH_3$ | |
| 4.50 | $SC_4H_9$-t | $SC_3H_7$-i | |
| 4.51 | $S(O)_2C_2H_5$ | $OCH_2CH=CH_2$ | |
| 4.52 | $OCH_2CH=CH_2$ | $OCH_3$ | |
| 4.53 | $OC_2H_5$ | $S(O)_2C_2H_5$ | |
| 4.54 | $S(O)_2C_2H_5$ | $OCH_2CH_2Cl$ | |
| 4.55 | $SCH_3$ | $OCH_3$ | Smp. 70 - 74 |
| 4.56 | $SC_4H_9$-t | $SC_4H_9$-t | |
| 4.57 | $OCH_2CH=CH_2$ | $OC_2H_5$ | |
| 4.58 | $SCH_3$ | $OC_2H_5$ | |
| 4.59 | $SC_2H_5$ | $SC_2H_5$ | |
| 4.60 | $SCH_3$ | $SCH_3$ | |
| 4.61 | $OCH_2CH=CH_2$ | $SCH_3$ | |
| 4.62 | $SCH_3$ | $SC_2H_5$ | |
| 4.63 | $SC_2H_5$ | $SC_3H_7$-i | |
| 4.64 | $OCH_2CH=CH_2$ | $SC_2H_5$ | |
| 4.65 | $SCH_3$ | $C_3H_7$-i | |
| 4.66 | $SC_2H_5$ | $SC_4H_9$-t | |

Tabelle 4 (Fortsetzung)

| Verb. Nr. | $R_a$ | $R_b$ | Physik. Konst. [°C] |
|---|---|---|---|
| 4.67 | $SCH_3$ | $SC_4H_9$-t | |
| 4.68 | $OCH_2CH{=}CH_2$ | $SC_3H_7$-i | |
| 4.69 | $OCH_2CH{=}CH_2$ | $OCH_2CH{=}CH_2$ | |
| 4.70 | $SCH_3$ | $OCH_2CH{=}CH_2$ | |
| 4.71 | $SC_2H_5$ | $OCH_2CH{=}CH_2$ | |
| 4.72 | $SCH_3$ | $OCH_2CH_2Cl$ | |
| 4.73 | $SC_2H_5$ | $OCH_2CH_2Cl$ | |
| 4.74 | $OCH(CH_3)CH_2CH_2CH_3$ | $SCH_3$ | |
| 4.75 | $SC_2H_5$ | $OC_2H_5$ | |
| 4.76 | $OCH(CH_3)CH_2CH_2CH_3$ | $SC_2H_5$ | |
| 4.77 | $OCH_2CH_2OCH_3$ | $SCH_3$ | |
| 4.78 | $SCH_3$ | $OCH_2CH_2OCH_3$ | |
| 4.79 | $SC_2H_5$ | $OCH_3$ | |
| 4.80 | $SC_2H_5$ | $OCH_2CH_2OCH_3$ | |
| 4.81 | $SCH_3$ | $OCH_2CH_2OC_2H_5$ | |
| 4.82 | $SC_2H_5$ | $SCH_3$ | |
| 4.83 | $SC_2H_5$ | $OCH_2CH_2OC_2H_5$ | |
| 4.84 | $SCH_2CH{=}CH_2$ | $OCH_3$ | Harz |

Tabelle 5 : Verbindungen der Formel

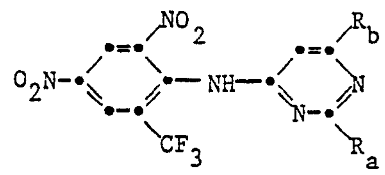

| Verb. Nr. | $R_a$ | $R_b$ | Physik. Konst. [°C] |
|---|---|---|---|
| 5.1 | $OCH_3$ | $OCH_3$ | |
| 5.2 | $SCH_3$ | $OCH_3$ | |
| 5.3 | $OCH_3$ | $OC_2H_5$ | |
| 5.4 | $SCH_3$ | $OC_2H_5$ | |
| 5.5 | $OCH_3$ | $SCH_3$ | |
| 5.6 | $SCH_3$ | $SCH_3$ | |
| 5.7 | $OCH_3$ | $SC_2H_5$ | |
| 5.8 | $SCH_3$ | $SC_2H_5$ | |
| 5.9 | $OCH_3$ | $OC_3H_7$-i | |
| 5.10 | $SCH_3$ | $OCH_2CH=CH_2$ | |
| 5.11 | $OCH_3$ | $S(O)_2CH_3$ | |
| 5.12 | $SC_2H_5$ | $OCH_3$ | |
| 5.13 | $SC_3H_7$-i | $OCH_3$ | |
| 5.14 | $OCH_3$ | $S(O)_2C_2H_5$ | |
| 5.15 | $SC_2H_5$ | $OC_2H_5$ | |
| 5.16 | $SC_3H_7$-i | $OC_2H_5$ | |
| 5.17 | $OC_2H_5$ | $OCH_3$ | |

Tabelle 5 (Fortsetzung)

| Verb. Nr. | $R_a$ | $R_b$ | Physik. Konst. [°C] |
|---|---|---|---|
| 5.18 | $SC_2H_5$ | $SCH_3$ | |
| 5.19 | $OC_2H_5$ | $OC_2H_5$ | |
| 5.20 | $SC_2H_5$ | $SC_2H_5$ | |
| 5.21 | $SC_2H_5$ | $OCH_2CH{=}CH_2$ | |
| 5.22 | $OC_2H_5$ | $SCH_3$ | |
| 5.23 | $OC_2H_5$ | $SC_2H_5$ | |
| 5.24 | $S(O)_2CH_3$ | $S(O)_2CH_3$ | |
| 5.25 | $SC_3H_7$-i | $SC_3H_7$-i | |
| 5.26 | $S(O)_2C_2H_5$ | $S(O)_2C_2H_5$ | |
| 5.27 | $OC_2H_5$ | $S(O)_2CH_3$ | |
| 5.28 | $S(O)_2C_3H_7$-i | $S(O)_2C_3H_7$-i | |
| 5.29 | $S(O)_2CH_3$ | $S(O)_2C_2H_5$ | |
| 5.30 | $S(O)CH_3$ | $S(O)CH_3$ | |
| 5.31 | $OC_2H_5$ | $S(O)_2C_2H_5$ | |
| 5.32 | $S(O)_2C_2H_5$ | $S(O)_2CH_3$ | |
| 5.33 | $OCH_2CH{=}CH_2$ | $SCH_3$ | |
| 5.34 | $OCH_2C{\equiv}CH$ | $SCH_3$ | |

Tabelle 6 : Verbindungen der Formel

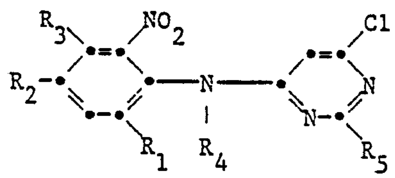

| Verb. Nr. | $R_1$ | $R_2$ | $R_3$ | $R_4$ | $R_5$ | Physik. Konst. [°C] |
|---|---|---|---|---|---|---|
| 6.1 | $NO_2$ | $CF_3$ | Cl | $CO-CH_3$ | Cl | |
| 6.2 | $CF_3$ | $NO_2$ | H | $CO-CH_3$ | Cl | |
| 6.3 | $NO_2$ | $CF_3$ | Cl | $CO-CH_2Cl$ | Cl | |
| 6.4 | $NO_2$ | $CF_3$ | Cl | $CO-CH_3$ | $OCH_3$ | |
| 6.5 | $CF_3$ | $NO_2$ | H | $CO-CH_2Cl$ | Cl | |
| 6.6 | $NO_2$ | $CF_3$ | Cl | $CO-CH_3$ | $SCH_3$ | Smp. 157-159 |
| 6.7 | $NO_2$ | $CF_3$ | Cl | $CO-CH_2OCH_3$ | Cl | |
| 6.8 | $CF_3$ | $NO_2$ | H | $CO-CH_3$ | $SCH_3$ | |
| 6.9 | $CF_3$ | $NO_2$ | H | $CO-CH_2OCH_3$ | Cl | |
| 6.10 | $NO_2$ | $CF_3$ | Cl | $CO-CH_2OC_2H_5$ | Cl | |
| 6.11 | $NO_2$ | $CF_3$ | Cl | $CO-CH_2OCH_3$ | $SCH_3$ | Smp. 98-102 |
| 6.12 | $NO_2$ | $CF_3$ | Cl | $CO-CH_2Cl$ | $SCH_3$ | Smp. 45-49 |

Tabelle 7 : Verbindungen der Formel

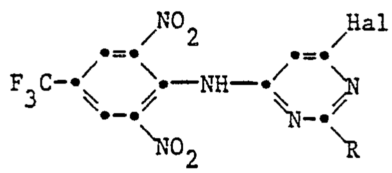

| Verb. Nr. | R | Hal | Physik. Konst. [°C] |
|---|---|---|---|
| 7.1 | Cl | Cl | Smp. 213-214 |
| 7.2 | $OCH_3$ | F | Harz |
| 7.3 | $OC_3H_7$-i | Cl | |
| 7.4 | $S(O)_2CH_3$ | Cl | |
| 7.5 | F | F | Harz |
| 7.6 | $OC_2H_5$ | Cl | |
| 7.7 | $OCH_3$ | Cl | Smp.212-214 |
| 7.8 | $OC_2H_5$ | F | Harz |
| 7.9 | $S(O)_2C_2H_5$ | Cl | |
| 7.10 | $OC_3H_7$-n | Cl | Smp.122,5-123,5 |
| 7.11 | $S(O)CH_3$ | Cl | |
| 7.12 | Br | Br | Harz |
| 7.13 | $OC_4H_9$-n | Cl | |
| 7.14 | S-Cyclohexyl | Cl | |
| 7.15 | J | J | |
| 7.16 | $OC_3H_7$-i | F | |

Tabelle 7 (Fortsetzung)

| Verb. Nr. | R | Hal | Physik. Konst. [°C] |
|---|---|---|---|
| 7.17 | $OC_4H_9$-t | Cl | |
| 7.18 | $S(O)C_2H_5$ | Cl | |
| 7.19 | $SCH_3$ | F | |
| 7.20 | $S(O)_2C_3H_7$-n | Cl | |
| 7.21 | $OCH_2CH(CH_3)C_2H_5$ | Cl | |
| 7.22 | $OCH(CH_3)C_2H_5$ | Cl | semikristallin |
| 7.23 | $OC_5H_{11}$-n | Cl | |
| 7.24 | O-Cyclohexyl | Cl | |
| 7.25 | $SC_2H_5$ | F | |
| 7.26 | $OC_6H_{11}$-n | Cl | |
| 7.27 | $OCH_3$ | Br | Harz |
| 7.28 | $OC_6H_{13}$-n | F | |
| 7.29 | $SC_4H_9$-t | F | |
| 7.30 | $OC_2H_5$ | Br | Harz |
| 7.31 | $OCH_2CH(CH_3)CH_2CH_2CH_3$ | Cl | |
| 7.32 | $SCH_3$ | Cl | Smp. 181-183 |
| 7.33 | $OC_3H_7$-i | Br | |
| 7.34 | $OCH_2CH(C_2H_5)_2$ | Cl | |
| 7.35 | $OCH_2CH_2OCH_3$ | F | |
| 7.36 | $SCH_3$ | Br | |

Tabelle 7 (Fortsetzung)

| Verb. Nr. | R | Hal | Physik. Konst. [°C] |
|---|---|---|---|
| 7.37 | $OC(CH_3)C{\equiv}CH$ | Cl | |
| 7.38 | $S(O)_2CH_3$ | F | |
| 7.39 | $SC_4H_9$-n | Cl | |
| 7.40 | $SC_2H_5$ | Cl | |
| 7.41 | $SCH_2CH{=}CH_2$ | Cl | |
| 7.42 | $OCH_2CF_3$ | Cl | semikristallin |
| 7.43 | $S(O)_2C_2H_5$ | F | |
| 7.44 | $SC_2H_5$ | Br | |
| 7.45 | $SC_3H_7$-n | Cl | |
| 7.46 | $SC_3H_7$-i | Br | |
| 7.47 | $SC_3H_7$-i | Cl | |
| 7.48 | $OC(CH_3)_2C{\equiv}CH$ | Cl | |
| 7.49 | $OCH_2C{\equiv}CH$ | Cl | semikristallin |
| 7.50 | $OCH_2CH_2Br$ | Br | |
| 7.51 | $S(O)_2CH_3$ | Br | |
| 7.52 | $S(O)_2C_6H_{13}$-n | F | |
| 7.53 | $SC_4H_9$-n | Cl | |
| 7.54 | $S(O)_2C_2H_5$ | Br | |
| 7.55 | $OCH_2CH{=}CH_2$ | Cl | semikristallin |
| 7.56 | $OCH_3$ | J | |

Tabelle 7 (Fortsetzung)

| Verb. Nr. | R | Hal | Physik. Konst. [°C] |
|---|---|---|---|
| 7.57 | $SC_4H_9$-t | Cl | |
| 7.58 | $OCH_2CH_2Br$ | Cl | Harz |
| 7.59 | $OCH_2CH(CH_3)_2$ | Cl | Smp. 138-142 |
| 7.60 | $OCH_2CH_2OC_2H_5$ | Cl | |
| 7.61 | $OC_3H_7$-i | J | |
| 7.62 | $SCH_3$ | J | |
| 7.63 | $OCH_2CH_2Cl$ | Cl | |
| 7.64 | $S(O)_2CH_3$ | J | |
| 7.65 | $OCH_2CH=CH_2$ | $OCH_2CH=CH_2$ | Oel |
| 7.66 | $OCH_2CH_2CH_2Cl$ | Cl | |
| 7.67 | $SC_2H_5$ | J | |
| 7.68 | $OCH_2CH=CH_2$ | J | |
| 7.69 | $OCH_2CH_2OCH_3$ | Cl | |
| 7.70 | $OCH_3$ | $OCH_3$ | Smp. 168-170 |

Formulierungsbeispiele für flüssige Wirkstoffe der Formel I (% = Gewichtsprozent)

| F1. Emulsions-Konzentrate | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenoyl-polyethylenglykolether (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff aus den Tabellen | 80% | 10% | 5% | 95% |
| Ethylenglykol-monomethyl-ether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Die Lösungen sind zur Anwendung in Form kleinster Tropfen geeignet.

| F3. Granulate | a) | b) |
|---|---|---|
| Wirkstoff aus den Tabellen | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| F4. Stäubemittel | a) | b) |
|---|---|---|
| Wirkstoff aus den Tabellen | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebauchsfertige Stäubemittel.

Formulierungsbeispiele für feste Wirkstoffe der Formel I (% = Gewichtsprozent)

| F5. Spritzpulver | a) | b) | c) |
|---|---|---|---|
| Wirkstoff aus den Tabellen | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

F6. Emulsions-Konzentrat

| | |
|---|---|
| Wirkstoff aus den Tabellen | 10% |
| Octylphenolpolyethylenglykolether (4-5 Mol Ethylenoxid) | 3% |
| Ca-Dodecylbenzolsulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff aus den Tabellen | 5% | 8% |
| Talkum | 95% | - |
| Kaolin | - | 92% |

Man erhält anwendungsfertige Stäubemittel, indem der Wirkstoff mit den Träger vermischt auf einer geeigneten Mühle vermahlen wird.

F8. Extruder Granulat

| | |
|---|---|
| Wirkstoff aus den Tabellen | 10% |
| N-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrum getrocknet.

F9. Umhüllungs-Granulat

| | |
|---|---|
| Wirkstoff aus den Tabellen | 3% |
| Polyethylenglykol (M G 200) | 3% |
| Kaolin | 94% |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

F10. Suspensions-Konzentrat

| | |
|---|---|
| Wirkstoff aus den Tabellen | 40% |
| Ethylenglykol | 10% |
| Nonylphenolpolyethylenglykolether (15 Mol Ethylenoxid) | 6% |
| N-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele :

Beispiel B1 : Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,02 % Aktivsubstanz) besprüht. Nach 24 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

b) Systemische Wirkung

Zu Weizenpflanzen wurden 5 Tage nach Aussaat eine aus Spritzpulver des Wirkstoffes hergestellte Spritzbrühe gegossen, (0,006 % Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

Verbindungen aus den Tabellen 1, 2 und 7, insbesondere Verbindungen der Gruppen Ib und Id zeigten gegen Puccinia-Pilze eine sehr gute Wirkung. Ungebhandelte aber infizierte Kontrollpflanzen zeigten einen Puccinia-Befall von 100 %. Unter anderen hemmten die Verbindungen 1.1, 1.4, 1.5, 1.8, 2.1, 2.2, 2.5, 2.6, 2.7, 2.8, 2.12, 2.27, 2.32, 2.40, 2.42, 2.49, 7.2, 7.5 und 7.32 den Puccinia-Befall auf 0 bis 5 %.

Beispiel B2 : Wirkung gegen Cersopora arachidicola auf Erdnusspflanzen

Residual-protektive Wirkung

10-15 cm hohe Erdnusspflanzen wurden mit einer aus Spritzpulver der Wirksubstanz hergestellten Spritzbrühe (0,006 Aktivsubstanz) besprüht und 48 Stunden später mit einer Konidiensuspension des Pilzes infiziert. Die infizierten Pflanzen wurden während 72 Stunden bei ca. 21 °C und hoher Luftfeuchtigkeit inkubiert und anschliessend bis zum Auftreten der typischen Blattflecken in einem Gewächshaus aufgestellt.

Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigten Erdnusspflanzen, die mit Wirkstoffen aus den Gruppen Ib und Id behandelt wurden, einen stark reduzierten Cercospora-Befall. So verhinderten die Verbindungen 1.1, 1.4, 1.5, 1.8, 1.63, 1.80, 2.2, 2.3, 2.32, 2.40, 7.1 und 7.32 in obigen Versuchen das Auftreten von Flecken fast vollständig (0-10 %).

Beispiel B3 : Wirkung gegen Erysiphae graminis auf Gerste

a) Residual-protektive Wirkung

Ca. 8 cm hohe Gerstenpflanzen wurden mit einer aus Spritzpulver des Wirkstoffes hergestellten Spritzbrühe (0,002 % Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

Verbindungen der Formel I, insbesondere die aus den Gruppen Ib und Id, zeigten gute Wirkung gegen Erysiphe-Pilze. Unbehandelte, aber infizierte Kontrollpflanzen zeigten einen Erysiphe-Befall von 100 %. Unter anderen Verbindungen aus den Tabellen hemmten den Pilzbefall bei Gerste auf weniger als 30 %, so z. B. die Verbindungen 7.1, 7.5 und 7.12.

Beispiel B4 : Residual-protektive Wirkung gegen Venturia inaequalis auf Apfeltrieben

Apfelstecklinge mit 10-20 cm langen Frischtrieben wurden mit einer aus Spritzpulver des Wirkstoffes

hergestellten Spritzbrühe (0,006 % Aktivsubstanz) besprüht. Nach 24 Stunden wurden die behandelten Pflanzen mit einer Konidiensuspension des Pilzes infiziert. Die Pflanzen wurden dann während 5 Tagen bei 90-100 % relativer Luftfeuchtigkeit inkubiert und während 10 weiteren Tagen in einem Gewächshaus bei 20-24 °C aufgestellt. Der Schorfbefall wurde 15 Tage nach der Infektion beurteilt. Verbindungen aus den Tabellen hemmten den Krankheitsbefall auf weniger als 25 %, so z. B. die Verbindungen 1.4, 1.5 und 1.12. Unbehandelte aber infizierte Kontrolltriebe wurden dagegen 100 %ig befallen.

Beispiel B5 : Wirkung gegen Botrytis cinerea auf Bohnen

Residual protektive Wirkung

Ca. 10 cm hohe Bohnen-Pflanzen wurden mit einer aus Spritzpulver des Wirkstoffes hergestellten Spritzbrühe (0,02 % Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95-100 % relativer Luftfeuchtigkeit und 21 °C erfolgte die Beurteilung des Pilzbefalls. Die Verbindungen aus der Tabelle hemmten in vielen Fällen die Pilzinfektion sehr stark. Bei einer Konzentration von 0,02 % erwiesen sich die Verbindungen aus den Tabellen 1, 2 und 7, so z. B. die Verbindungen 1.1, 1.4, 1.5, 1.8, 1,12, 7.1 und 7.32 als voll wirksam. Der Krankheitsbefall lag bei 0 bis 8 %. Der Botrytis-Befall unbehandelter ber infizierter Bohnenpflanzen betrug 100 %.

Beispiel B6 : Wirkung gegen Phytophthora infestans auf Tomatenpflanzen

a) Residual-protektive Wirkung

Tomatenpflanzen wurden nach 3-wöchiger Anzucht mit einer aus Spritzpulver des Wirkstoffes hergestellten Spritzbrühe (0,06 % Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20 °C.

b) Systemische Wirkung

Zu Tomatenpflanzen wurde nach 3-wöchiger Anzucht eine aus Spritzpulver des Wirkstoffes hergestellte Spritzbrühe gegossen (0,06 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20 °C.

Insbesondere Verbindungen aus den Untergruppen Ib und Id zeigten in obigen Versuchen eine sehr gute systemische Wirkung, so z. B. die Verbindungen Nr. 1.1, 1.2, 1.4, 1.5, 1.7, 1.8, 1.12, 1.16, 1.20, 1.37, 1.49, 1.57, 1.74, 1.80, 1.84, 2.1, 2.2, 2.5, 2.6, 2.7, 2.8, 2.27, 2.32, 2.49, 2.55, 4.4, 7.7 und 7.32. Gegenüber unbehandelten Kontrollpflanzen (100 % Befall) bewirkten diese Verbindungen eine fast vollständige Unterdrückung des Pilzbefalls (0 bis 5 %).

Beispiel B7 : Wirkung gegen Plasmapora viticola auf Reben

a) Residual-protektive Wirkung

Im 4-5 Blattstadium wurden Rebensämlinge mit einer aus Spritzpulver des Wirkstoffes hergestellten Spritzbrühe (0,06 % Aktivsubstanz) besprüht. Nach 24 Stunden wurden die behandelten Pflanzen mit einer Sporangiensuspension des Pilzes infiziert. Nach einer Inkubation während 6 Tagen bei 95-100 % relativer Luftfeuchtigkeit und 20 °C wurde der Pilzbefall beurteilt.

b) Residual-kurative Wirkung

Im 4-5 Blattstadium wurden Rebensämlinge mit einer Sporangiensuspension des Pilzes infiziert. Nach einer Inkubation während 24 Stunden in einer Feuchtkammer bei 95-100 % relativer Luftfeuchtigkeit und 20 °C wurden die infizierten Pflanzen getrocknet und mit einer aus Spritzpulver des Wirkstoffes hergestellten Spritzbrühe (0,06 % Aktivsubstanz) besprüht. Nach dem Antrocknen des Spritzbelages wurden die behandelten Pflanzen wieder in die Feuchtkammer gebracht. Die Beurteilung des Pilzbefalls erfolgte 6 Tage nach der Infektion.

Verbindungen aus den Tabellen 1, 2 und 7 zeigten gegen Plasmopara viticola auf Regen eine sehr gute fungizide Wirkung, insbesondere die Wirkstoffe Nr. 1.1, 1.4, 1.5, 1.8 und 7.32 bewirkten eine vollständige Minderung des Pilzbefalls (0 bis 5 %).

Beispiel B8 : Wirkung gegen Piricularia oryzae auf Reispflanzen

Residual-protektive Wirkung

Reispflanzen wurden nach zweiwöchiger Anzucht mit einer aus Spritzpulver des Wirkstoffes hergestellten Spritzbrühe (0,02 % Aktivsubstanz) besprüht. Nach 48 Stunden wurden die behandelten Pflanzen mit einer Konidiensuspension des Pilzes infiziert. Nach 5 Tagen Inkubation bei 95-100 % relativer Luftfeuchtigkeit und 24 °C wurde der Pilzbefall beurteilt.

Reisplflanzen, die mit einer Spritzbrühe behandelt worden sind, die als Aktivsubstanz eine der Verbindungen 1.1, 1.4, 1.5, 1.8 und 7.32 enthielt, zeigten im Vergleich zu unbehandelten Kontrollpflanzen (100 % Befall) weniger als 10 % Pilzbefall.

**Patentansprüche** (für die Vertragsstaaten : BE, CH, LI, DE, FR, IT, LU, NL, SE)

1. Verbindungen der allgemeinen Formel I

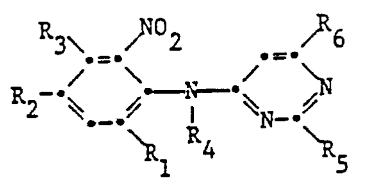

(I)

worin

$R_1$ für $NO_2$ oder $CF_3$ steht ;
$R_2$ für $NO_2$ oder $CF_3$ steht ;
$R_3$ Wasserstoff oder Halogen bedeutet ;
$R_4$ für Wasserstoff oder die Gruppe —C(O)$R_7$ steht, wobei $R_7$ unsubstituiertes oder durch Halogen, $C_1$-$C_3$-Alkoxy oder $C_1$-$C_3$-Alkylthio substituiertes $C_1$-$C_4$-Alkyl bedeutet ;
$R_5$ und $R_6$ unabhängig voneinander für Halogen, $C_1$-$C_6$-Alkoxy, durch Halogen oder $C_1$-$C_3$-Alkoxy substituiertes $C_1$-$C_6$-Alkyl, durch Halogen oder $C_1$-$C_3$-Alkoxy substituiertes $C_1$-$C_6$-Alkoxy, $C_1$-$C_6$-Alkylthio, durch Halogen oder $C_1$-$C_3$-Alkoxy substituiertes $C_1$-$C_6$-Alkylthio, $C_3$-$C_6$-Cycloalkoxy, $C_3$-$C_6$-Cycloalkylthio, ($C_2$-$C_5$-Alkenyl)-methoxy, ($C_2$-$C_5$-Alkenyl)-methylthio, ($C_2$-$C_5$-Alkinyl)-methoxy, $C_1$-$C_6$-Alkylsulfoxyl, $C_1$-$C_6$-Alkylsulfonyl, $C_1$-$C_6$-Sulfonyloxy, $C_3$-$C_6$-Alkenylsulfoxyl, $C_3$-$C_6$-Alkenylsulfonyl, $C_3$-$C_6$-Cycloalkylsulfoxyl oder für $C_3$-$C_6$-Cycloalkylsulfonyl stehen.

2. Verbindungen der Formel I nach Anspruch 1, dadurch gekennzeichnet, dass $R_1$ für $NO_2$, $R_2$ für $CF_3$, $R_3$ für Chlor und $R_4$ für Wasserstoff stehen, $R_5$ Halogen, $C_1$-$C_6$-Alkoxy, durch Chlor oder Brom substituiertes $C_1$-$C_3$-Alkyl, durch $C_1$-$C_3$-Alkoxy substituiertes $C_1$-$C_3$-Alkoxy, $C_1$-$C_6$-Alkylthio, Cyclopentyloxy, Cyclohexyloxy, Cyclopentylthio, Cyclohexylthio, ($C_2$-$C_5$-Alkenyl)-methoxy, ($C_2$-$C_5$-Alkenyl)-methylthio, ($C_2$-$C_5$-Alkinyl)-methoxy, $C_1$-$C_3$-Alkylsulfoxyl, $C_1$-$C_6$-Alkylsulfonyl, $C_1$-$C_3$-Alkylsulfonyloxy oder Cyclohexylsulfonyl bedeutet ; und $R_6$ für Halogen steht.

3. Verbindungen der Formel I nach Anspruch 1, worin $R_1$ für $NO_2$, $R_2$ für $CF_3$, $R_3$ für Chlor oder Brom stehen, $R_5$ Halogen, $C_1$-$C_4$-Alkoxy, durch Chlor oder Brom substituiertes $C_1$-$C_3$-Alkyl, durch Methoxy substituiertes $C_1$-$C_2$-Alkoxy, $C_1$-$C_4$-Alkylthio, ($C_2$-$C_3$-Alkenyl)-methoxy, ($C_2$-$C_3$-Alkinyl)-methoxy oder $C_1$-$C_3$-Alkylsulfonyl bedeutet ; und $R_6$ für Fluor, Chlor oder Brom steht.

4. Verbindungen der Formel I nach Anspruch 1, dadurch gekennzeichnet, dass $R_1$ für $CF_3$, $R_2$ für $NO_2$, $R_3$ für Wasserstoff und $R_4$ für Wasserstoff stehen, $R_5$ Halogen, $C_1$-$C_6$-Alkoxy, durch Chlor oder Brom substituiertes $C_1$-$C_3$-Alkyl, durch $C_1$-$C_3$-Alkoxy substituiertes $C_1$-$C_3$-Alkoxy, $C_1$-$C_6$-Alkylthio, Cyclopentyloxy, Cyclohexyloxy, Cyclopentylthio, Cyclohexylthio, ($C_2$-$C_5$-Alkenyl)-methoxy, ($C_2$-$C_5$-Alkenyl)-methylthio, ($C_2$-$C_5$-Alkinyl)-methoxy, $C_1$-$C_3$-Alkylsulfoxyl, $C_1$-$C_6$-Alkylsulfonyl, $C_1$-$C_3$-Alkylsulfonyloxy oder Cyclohexylsulfonyl bedeutet ; und $R_6$ für Halogen steht.

5. Verbindungen der Formel I nach Anspruch 1, worin $R_1$ für $CF_3$, $R_2$ für $NO_2$, $R_3$ für Chlor oder Brom stehen, $R_5$ Halogen, $C_1$-$C_4$-Alkoxy, durch Chlor oder Brom substituiertes $C_1$-$C_3$-Alkyl, durch Methoxy substituiertes $C_1$-$C_2$-Alkoxy, $C_1$-$C_4$-Alkylthio, ($C_2$-$C_3$-Alkenyl)-methoxy, ($C_2$-$C_3$-Alkinyl)-methoxy oder $C_1$-$C_3$-Alkylsulfonyl bedeutet ; und $R_6$ für Fluor, Chlor oder Brom steht.

6. Verbindungen der Formel I nach Anspruch 1, dadurch gekennzeichnet, dass $R_1$ für $NO_2$, $R_2$ für $CF_3$, $R_3$ für Halogen und $R_4$ für Wasserstoff stehen, $R_5$ für $C_1$-$C_3$-Alkoxy, $C_1$-$C_3$-Alkylthio, $C_1$-$C_3$-Alkylsulfoxyl oder $C_1$-$C_3$-Alkylsulfonyl bedeutet und $R_6$ für Halogen steht.

7. Verbindungen der Formel I nach Anspruch 1, dadurch gekennzeichnet, dass $R_1$ für $NO_2$, $R_2$ für $CF_3$, $R_3$ für Chlor und $R_4$ für Wasserstoff stehen, während $R_5$ und $R_6$ unabhängig voneinander Halogen, $C_1$-$C_6$-Alkoxy, durch Chlor oder Brom substituiertes $C_1$-$C_3$-Alkyl, durch $C_1$-$C_3$-Alkoxy substituiertes $C_1$-$C_3$-Alkoxy, $C_1$-$C_6$-Alkylthio, Cuclopentyloxy, Cyclohexyloxy, Cyclopentylthio, Cyclohexylthio, ($C_2$-$C_5$-Alkenyl)-methoxy, ($C_2$-Alkenyl)-methylthio, ($C_2$-$C_5$-Alkinyl)-methoxy, $C_1$-$C_3$-Alkylsulfoxyl, $C_1$-$C_6$-Alkylsulfonyl, $C_1$-$C_3$-Alkylsulfonyloxy oder Cyclohexylsulfonyl bedeuten.

8. Verbindungen der Formel I nach Anspruch 1, dadurch gekennzeichnet, dass $R_1$ für $CF_3$, $R_2$ für $NO_2$, $R_3$ für Wasserstoff und $R_4$ für Wasserstoff stehen und $R_5$ und $R_6$ unabhängig voneinander $C_1$-$C_3$-Alkoxy, $C_1$-$C_3$-Alkylthio, ($C_2$-Alkenyl)-methoxy, $C_1$-$C_3$-Alkenylsulfoxyl oder $C_1$-$C_3$-Alkenylsulfonyl bedeuten.

9. Verbindungen der Formel I nach Anspruch 1, dadurch gekennzeichnet, dass $R_1$ für $CF_3$ oder $NO_2$ steht ; $R_2$ $CF_3$ oder $NO_2$ bedeutet ; $R_3$ Wasserstoff oder Chlor bedeutet ; $R_4$ für die Gruppe C(O)—$R_7$ steht, worin $R_7$ $C_1$-$C_3$-Alkyl, durch Halogen substituiertes $C_1$-$C_3$-Alkyl oder durch $C_1$-$C_3$-Alkoxy substituiertes $C_1$-$C_3$-Alkyl bedeutet ; $R_5$ für Halogen, $C_1$-$C_3$-Alkoxy oder $C_1$-$C_3$-Alkylthio steht und $R_6$ Chlor bedeutet.

10. Verbindungen der Formel I nach Anspruch 1, dadurch gekennzeichnet, dass $R_1$ für $NO_2$, $R_2$ für $CF_3$, $R_3$ für Wasserstoff und $R_4$ für Wasserstoff stehen, $R_5$ Halogen, $C_1$-$C_6$-Alkoxy, durch Chlor oder Brom substituiertes $C_1$-$C_3$-Alkyl, durch $C_1$-$C_3$-Alkoxy substituiertes $C_1$-$C_3$-Alkoxy, $C_1$-$C_6$-Alkylthio, Cyclopentyloxy, Cyclohexyloxy, Cyclopentylthio, Cyclohexylthio, ($C_2$-$C_5$-Alkenyl)-methoxy, ($C_2$-$C_5$-Alkenyl)-methylthio, ($C_2$-$C_5$-Alkinyl)-methoxy, $C_1$-$C_3$-Alkylsulfoxyl, $C_1$-$C_6$-Alkylsulfonyl, $C_1$-$C_3$-Alkylsulfonyloxy oder Cyclohexylsulfonyl bedeutet ; und $R_6$ für Halogen steht.

11. Verbindungen der Formel I nach Anspruch 1, ausgewählt aus der Reihe :
N-(3'-Chlor-2',6'-dinitro-4'-trifluormethylphenyl)-4-amino-2,6-dichlorpyrimidin ;
N-(2',4'-Dinitro-6'-trifluormethylphenyl)-4-amino-2,6-dichlorpyridin ;
N-(3'-Chlor-2',6'-dinitro-4'-trifluormethylphenyl)-4-amino-6-chlor-2-methylmercaptopyrimidin ;
N-(2',6'-Dinitro-4'-trifluormethylphenyl)-4-amino-6-chlor-2-methylmercapto-pyridin ;
N-(3'-Chlor-2',6'-dinitro-4'-trifluormethylphenyl)-4-amino-6-chlor-2-methoxy-pyrimidin ;
N-(3'-Chlor-2',6'-dinitro-4'-trifluormethylphenyl)-4-amino-6-chlor-2-ethoxy-pyrimidin ;
N-(3'-Chlor-2',6'-dinitro-4'-trifluormethylphenyl)-4-amino-6-chlor-2-ethylmercapto-pyrimidin ;
N-(3'-Chlor-2',6'-dinitro-4'-trifluormethylphenyl)-4-amino-6-chlor-2-sek.-butylmercapto-pyrimidin ;
N-(2',6'-Dinitro-4'-trifluormethylphenyl)-4-amino-2,6-dichlor-pyrimidin ;
N-(2',4'-Dinitro-6'-trifluormethylphenyl)-4-amino-2-methoxy-6-chlor-pyrimidin ;
N-(2',4'-Dinitro-6'-trifluormethylphenyl)-4-amino-2-ethoxy-6-chlor-pyrimidin.

12. Verfahren zur Herstellung von Verbindungen der Formel I, dadurch gekennzeichnet, dass man eine Verbindung der Formel II

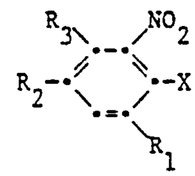

(II)

in Gegenwart einer Base mit einem Pyrimidin-Derivat der Formel III

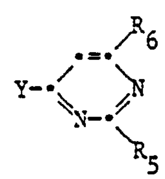

(III)

zu einer Verbindung der Formel I'

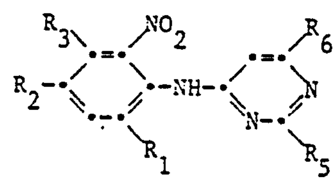

(I')

umsetzt, und letztere zur Herstellung N-acylierter Derivate mit einem reaktionsfähigen Derivat der Carbonsäure der Formel IV

$$R_7COOH \qquad (IV)$$

N-acyliert, wobei die Substituenten $R_1$ bis $R_7$ die unter Formel I angegebenen Bedeutungen haben und X und Y für $NH_2$ oder Halogen stehen, wobei in dem Fall, in dem X für Halogen steht, Y $NH_2$ bedeutet und in dem Fall in dem X für $NH_2$ steht, Y Halogen bedeutet.

13. Mittel zur Bekämpfung oder Verhütung eines Befalls durch Mikroorganismen, dadurch gekennzeichnet, dass es als mindestens eine aktive Komponente eine Verbindung der Formel I gemäss Anspruch 1 enthält.

14. Mittel gemäss Anspruch 13, dadurch gekennzeichnet, dass es als mindestens eine aktive Komponente eine Verbindung der Formel I gemäss Anspruch 2 enthält.

15. Mittel zur Bekämpfung von Mikroorganismen gemäss Anspruch 13, dadurch gekennzeichnet, dass es als mindestens eine aktive Komponente eine Verbindung der Formel I gemäss einem der Ansprüche 3 bis 11 enthält.

16. Mittel nach Anspruch 13, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

17 Mittel nach Anspruch 16, dadurch gekennzeichnet, dass es 0,1 bis 95 % eines Wirkstoffs der Formel I, 99,8 bis 5 % eines festen oder flüssigen Zusatzstoffes und 0,1 bis 25 % eines Tensids enthält.

18. Verfahren zur Bekämpfung oder Verhütung eines Befalls von Kulturpflanzen durch phytopathogene Mikroorganismen, dadurch gekennzeichnet, dass man eine gemäss Anspruch 1 definierte Verbindung der Formel I auf die Pflanze oder deren Standort appliziert.

**Patentansprüche** (für den Vertragsstaat AT)

1. Fungizides Mittel, enthaltend neben üblichen Träger- und Hilfsstoffen eine Verbindung der allgemeinen Formel I

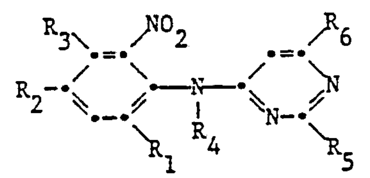

(I)

worin

$R_1$ für $NO_2$ oder $CF_3$ steht ;

$R_2$ für $NO_2$ oder $CF_3$ steht ;

$R_3$ Wasserstoff oder Halogen bedeutet ;

$R_4$ für Wasserstoff oder die Gruppe —C(O)$R_7$ steht, wobei $R_7$ unsubstituiertes oder durch Halogen, $C_1$-$C_3$-Alkoxy oder $C_1$-$C_3$-Alkylthio substituiertes $C_1$-$C_4$-Alkyl bedeutet ;

$R_5$ und $R_6$ unabhängig voneinander für Halogen, $C_1$-$C_6$-Alkoxy, durch Halogen oder $C_1$-$C_3$-Alkoxy substituiertes $C_1$-$C_6$-Alkyl, durch Halogen oder $C_1$-$C_3$-Alkoxy substituiertes $C_1$-$C_6$-Alkoxy, $C_1$-$C_6$-Alkylthio, durch Halogen oder $C_1$-$C_3$-Alkoxy substituiertes $C_1$-$C_6$-Alkylthio, $C_3$-$C_6$-Cycloalkoxy, $C_3$-$C_6$-Cycloalkylthio, ($C_2$-$C_5$-Alkenyl)-methoxy, ($C_2$-$C_5$-Alkenyl)-methylthio, ($C_2$-$C_5$-Alkinyl)-methoxy, $C_3$-$C_6$-Alkinyloxy, $C_1$-$C_6$-Alkylsulfoxyl, $C_1$-$C_6$-Alkylsulfonyl, $C_1$-$C_6$-Alkyl-sulfonyloxy, $C_3$-$C_6$-Alkenylsulfoxyl, $C_3$-$C_6$-Alkenylsulfonyl, $C_3$-$C_6$-Cycloalkylsuloxyl oder für $C_3$-$C_6$-Cycloalkylsulfonyl stehen.

2. Mittel nach Anspruch 1, enthaltend eine Verbindung der Formel I, dadurch gekennzeichnet, dass $R_1$ für $NO_2$, $R_2$ für $CF_3$, $R_3$ für Chlor und $R_4$ für Wasserstoff stehen, $R_5$ Halogen, $C_1$-$C_6$-Alkoxy, durch Chlor oder Brom substituiertes $C_1$-$C_3$-Alkyl, durch $C_1$-$C_3$-Alkoxy substituiertes $C_1$-$C_3$-Alkoxy, $C_1$-$C_6$-Alkylthio, Cyclopentyloxy, Cyclohexyloxy, Cyclopentylthio, Cyclohexylthio, ($C_2$-$C_5$-Alkenyl)-methoxy, ($C_2$-$C_5$-Alkenyl)-methylthio, ($C_2$-$C_5$-Alkinyl)-methoxy, $C_1$-$C_3$-Alkylsulfoxyl, $C_1$-$C_6$-Alkylsulfonyl, $C_1$-$C_3$-Alkylsulfonyloxy oder Cyclohexylsulfonyl bedeutet ; und $R_6$ für Halogen steht.

3. Mittel nach Anspruch 1, enthaltend eine Verbindung der Formel I, worin $R_1$ für $NO_2$, $R_2$ für $CF_3$, $R_3$ für Chlor oder Brom stehen, $R_5$ Halogen, $C_1$-$C_4$-Alkoxy, durch Chlor oder Brom substituiertes $C_1$-$C_3$-Alkyl, durch Methoxy substituiertes $C_1$-$C_2$-Alkoxy, $C_1$-$C_4$-Alkylthio, ($C_2$-$C_3$-Alkenyl)-methoxy, ($C_2$-$C_3$-Alkinyl)-methoxy oder $C_1$-$C_3$-Alkylsulfonyl bedeutet ; und $R_6$ für Fluor, Chlor oder Brom steht.

4. Mittel nach Anspruch 1, enthaltend eine Verbindung der Formel I, dadurch gekennzeichnet, dass $R_1$ für $CF_3$, $R_2$ für $NO_2$, $R_3$ für Wasserstoff und $R_4$ für Wasserstoff stehen, $R_5$ Halogen, $C_1$-$C_6$-Alkoxy, durch Chlor oder Brom substituiertes $C_1$-$C_3$-Alkyl, durch $C_1$-$C_3$-Alkoxy substituiertes $C_1$-$C_3$-Alkoxy, $C_1$-$C_6$-Alkylthio, Cyclopentyloxy, Cyclohexyloxy, Cyclopentylthio, Cyclohexylthio, ($C_2$-$C_5$-Alkenyl)-methoxy, ($C_2$-$C_5$-Alkenyl)-methylthio, ($C_2$-$C_5$-Alkinyl)-methoxy, $C_1$-$C_3$-Alkylsulfoxyl, $C_1$-$C_6$-Alkylsulfonyl, $C_1$-$C_3$-Alkylsulfonyloxy oder Cyclohexylsulfonyl bedeutet ; und $R_6$ für Halogen steht.

5. Mittel nach Anspruch 1, enthaltend eine Verbindung der Formel I, worin $R_1$ für $CF_3$, $R_2$ für $NO_2$, $R_3$ für Chlor oder Brom stehen, $R_5$ Halogen, $C_1$-$C_4$-Alkoxy, durch Chlor oder Brom substituiertes $C_1$-$C_3$-Alkyl, durch Methoxy substituiertes $C_1$-$C_2$-Alkoxy, $C_1$-$C_4$-Alkylthio, ($C_2$-$C_3$-Alkenyl)-methoxy, $C_2$-$C_3$-Alkinyl)-methoxy oder $C_1$-$C_3$-Alkylsulfonyl bedeutet ; und $R_6$ für Fluor, Chlor oder Brom steht.

6. Mittel nach Anspruch 1, enthaltend eine Verbindung der Formel I, dadurch gekennzeichnet, dass $R_1$ für $NO_2$, $R_2$ für $CF_3$, $R_3$ für Halogen und $R_4$ für Wasserstoff stehen, $R_5$ für $C_1$-$C_3$-Alkoxy, $C_1$-$C_3$-Alkylthio, $C_1$-$C_3$-Alkylsulfoxyl oder $C_1$-$C_3$-Alkylsulfonyl bedeutet und $R_6$ für Halogen steht.

7. Mittel nach Anspruch 1, enthaltend eine Verbindung der Formel I, dadurch gekennzeichnet, dass $R_1$ für $NO_2$, $R_2$ für $CF_3$, $R_3$ für Chlor und $R_4$ für Wasserstoff stehen, während $R_5$ und $R_6$ unabhängig voneinander Halogen, $C_1$-$C_6$-Alkoxy, durch Chlor oder Brom substituiertes $C_1$-$C_3$-Alkyl, durch $C_1$-$C_3$-Alkoxy substituiertes $C_1$-$C_3$-Alkoxy, $C_1$-$C_6$-Alkylthio, Cyclopentyloxy, Cyclohexyloxy, Cyclopentylthio, Cyclohexylthio, ($C_2$-$C_5$-Alkenyl)-methoxy, ($C_2$-Alkenyl)-methylthio, ($C_2$-$C_5$-Alkinyl)-methoxy, $C_1$-$C_3$-Alkylsulfoxyl, $C_1$-$C_6$-Alkylsulfonyl, $C_1$-$C_3$-Alkylsulfonyloxy oder Cyclohexylsulfonyl bedeuten.

8. Mittel nach Anspruch 1, enthaltend eine Verbindung der Formel I, dadurch gekennzeichnet, dass $R_1$ für $CF_3$, $R_2$ für $NO_2$, $R_3$ für Wasserstoff und $R_4$ für Wasserstoff stehen und $R_5$ und $R_6$ unabhängig voneinander $C_1$-$C_3$-Alkoxy, $C_1$-$C_3$-Alkylthio, ($C_2$-Alkenyl)-methoxy, $C_1$-$C_3$-Alkenylsulfoxyl oder $C_1$-$C_3$-Alkenylsulfonyl bedeuten.

9. Mittel nach Anspruch 1, enthaltend eine Verbindung der Formel I, dadurch gekennzeichnet, dass

$R_1$ für $CF_3$ oder $NO_2$ steht ; $R_2$ $CF_3$ oder $NO_2$ bedeutet ; $R_3$ Wasserstoff oder Chlor bedeutet ; $R_4$ für die Gruppe C(O)—$R_7$ steht, worin $R_7$ $C_1$-$C_3$-Alkyl, durch Halogen substituiertes $C_1$-$C_3$-Alkyl oder durch $C_1$-$C_3$-Alkoxy substituiertes $C_1$-$C_3$-Alkyl bedeutet ; $R_5$ für Halogen, $C_1$-$C_3$-Alkoxy oder $C_1$-$C_3$-Alkylthio steht und $R_6$ Chlor bedeutet.

10. Mittel nach Anspruch 1, enthaltend eine Verbindung der Formel I, dadurch gekennzeichnet, dass $R_1$ für $NO_2$, $R_2$ für $CF_3$, $R_3$ für Wasserstoff und $R_4$ für Wasserstoff stehen, $R_5$ Halogen, $C_1$-$C_6$-Alkoxy, durch Chlor oder Brom substituiertes $C_1$-$C_3$-Alkyl, durch $C_1$-$C_3$-Alkoxy substituiertes $C_1$-$C_3$-Alkoxy, $C_1$-$C_6$-Alkylthio, Cyclopentyloxy, Cyclohexyloxy, Cyclopentylthio, Cyclohexylthio, ($C_2$-$C_5$-Alkenyl)-methoxy, ($C_2$-$C_5$-Alkenyl)-methylthio, ($C_2$-$C_5$-Alkinyl)-methoxy, $C_1$-$C_3$-Alkylsulfoxyl, $C_1$-$C_6$-Alkylsulfonyl, $C_1$-$C_3$-Alkylsulfonyloxy oder Cyclohexylsulfonyl bedeutet ; und $R_6$ für Halogen steht.

11. Mittel nach Anspruch 1, enthaltend eine Verbindung der Formel I, ausgewählt aus der Reihe :
N-(3'-Chlor-2',6'-dinitro-4'-trifluormethylphenyl)-4-amino-2,6-dichlorpyrimidin :
N-(2',4'-Dinitro-6'-trifluormethylphenyl)-4-amino-2,6-dichlorpyridin ;
N-(3'-Chlor-2',6'-dinitro-4'-trifluormethylphenyl)-4-amino-6-chlor-2-methylmercaptopyrimidin ;
N-(2',6'-Dinitro-4'-trifluormethylphenyl)-4-amino-6-chlor-2-methylmercapto-pyridin ;
N-(3'-Chlor-2',6'-dinitro-4'-trifluormethylphenyl)-4-amino-6-chlor-2-methoxy-pyrimidin :
N-(3'-Chlor-2',6'-dinitro-4'-trifluormethylphenyl)-4-amino-6-chlor-2-ethoxy-pyrimidin ;
N-(3'-Chlor-2',6'-dinitro-4'-trifluormethylphenyl)-4-amino-6-chlor-2-ethylmercapto-pyrimidin ;
N-(3'-Chlor-2',6'-dinitro-4'-trifluormethylphenyl)-4-amino-6-chlor-2-sek.-butylmercapto-pyrimidin ;
N-(2',6'-Dinitro-4'-trifluormethylphenyl)-4-amino-2,6-dichlorpyrimidin ;
N-(2',4'-Dinitro-6'-trifluormethylphenyl)-4-amino-2-methoxy-6-chlorpyrimidin ;
N-(2',4'-Dinitro-6'-trifluormethylphenyl)-4-amino-2-ethoxy-6-chlorpyrimidin.

12. Verfahren zur Herstellung von Verbindungen der Formel I, dadurch gekennzeichnet, dass man eine Verbindung der Formel II

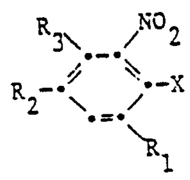

(II)

in Gegenwart einer Base mit einem Pyrimidin-Derivat der Formel III

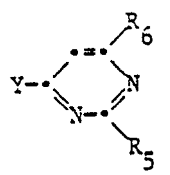

(III)

zu einer Verbindung der Formel I'

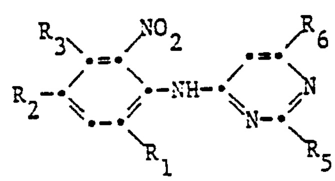

(I')

umsetzt, und letztere zur Herstellung N-acylierter Derivate mit einem reaktionsfähigen Derivat der Carbonsäure der Formel IV

$$R_7COOH \quad (IV)$$

N-acyliert, wobei die Substituenten $R_1$ bis $R_7$ die unter Formel I angegebenen Bedeutungen haben und X und Y für $NH_2$ oder Halogen stehen, wobei in dem Fall, in dem X für Halogen steht, Y $NH_2$ bedeutet und in dem Fall in dem X für $NH_2$ steht, Y Halogen bedeutet.

13. Verfahren zur Bekämpfung oder Verhütung eines Befalls von Kulturpflanzen durch phytopathogene Mikroorganismen, dadurchgekennzeichnet, dass man eine gemäss Anspruch 1 definierte Verbindung der Formel I auf die Pflanze oder deren Standort appliziert.

**Claims** (for the Contracting States : BE, CH, LI, DE, FR, IT, LU, NL, SE)

1. Compounds of the general formula I

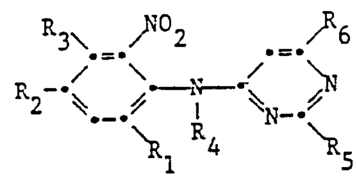

(I)

wherein

$R_1$ is $NO_2$ or $CF_3$ ;

$R_2$ is $NO_2$ or $CF_3$ ;

$R_3$ is hydrogen or halogen ;

$R_4$ is hydrogen of the —C(O)$R_7$ group, wherein $R_7$ is $C_1$-$C_4$ alkyl which is unsubstituted or substituted by halogen, $C_1$-$C_3$ alkoxy or by $C_1$-$C_3$ alkylthio ;

$R_5$ and $R_6$ are each independently halogen, $C_1$-$C_6$ alkoxy, $C_1$-$C_6$ alkyl which is substituted by halogen or by $C_1$-$C_3$ alkoxy, $C_1$-$C_6$ alkoxy which is substituted by halogen or by $C_1$-$C_3$ alkoxy, $C_1$-$C_6$ alkylthio, $C_1$-$C_6$ alkylthio which is substituted by halogen or by $C_1$-$C_3$ alkoxy, $C_3$-$C_6$ cycloalkoxy, $C_3$-$C_6$ cycloalkylthio, ($C_2$-$C_5$ alkenyl)-methoxy, ($C_2$-$C_5$ alkenyl)-methylthio, ($C_2$-$C_5$ alkynyl)-methoxy, $C_1$-$C_6$ alkylsulfoxyl, $C_1$-$C_6$ alkylsulfonyl, $C_1$-$C_6$ sulfonyloxy, $C_3$-$C_6$ alkenylsulfoxyl, $C_3$-$C_6$ alkenylsulfonyl, $C_3$-$C_6$ cycloalkylsulfoxyl or $C_3$-$C_6$ cycloalkylsulfonyl.

2. Compounds of the formula I according to claim 1, characterised in that $R_1$ is $NO_2$, $R_2$ is $CF_3$, $R_3$ is chlorine and $R_4$ is hydrogen, $R_5$ is halogen, $C_1$-$C_6$ alkoxy, chlorine- or bromine-substituted $C_1$-$C_3$ alkyl, $C_1$-$C_3$ alkoxy-substituted $C_1$-$C_3$ alkoxy, $C_1$-$C_6$ alkylthio, cyclopentyloxy, cyclohexyloxy, cyclopentylthio, cyclohexylthio, ($C_2$-$C_5$ alkenyl)-methoxy, ($C_2$-$C_5$ alkenyl)-methylthio, ($C_2$-$C_5$ alkynyl)-methoxy, $C_1$-$C_3$ alkylsulfoxyl, $C_1$-$C_6$ alkylsulfonyl, $C_1$-$C_3$ alkylsulfonyloxy or cyclohexylsulfonyl ; and $R_6$ is halogen.

3. Compounds of the formula I according to claim 1, wherein $R_1$ is $NO_2$, $R_2$ is $CF_3$, $R_3$ is chlorine or bromine, $R_5$ is halogen, $C_1$-$C_4$ alkoxy, chlorine- or bromine-substituted $C_1$-$C_3$ alkyl, methoxy-substituted $C_1$-$C_2$ alkoxy, $C_1$-$C_4$ alkylthio, ($C_2$-$C_3$ alkenyl)-methoxy, ($C_2$-$C_3$ alkynyl)-methoxy or $C_1$-$C_3$ alkylsulfonyl ; and $R_6$ is fluorine, chlorine or bromine.

4. Compounds of the formula I according to claim 1, characterised in that $R_1$ is $CF_3$, $R_2$ is $NO_2$, $R_3$ is hydrogen and $R_4$ is hydrogen, $R_5$ is halogen, $C_1$-$C_6$-alkoxy, chlorine- or bromine-substituted $C_1$-$C_3$ alkyl, $C_1$-$C_3$ alkoxy-substituted $C_1$-$C_3$ alkoxy, $C_1$-$C_6$ alkylthio, cyclopentyloxy, cyclohexyloxy, cyclopentylthio, cyclohexylthio, ($C_2$-$C_5$ alkenyl)-methoxy, ($C_2$-$C_5$ alkynyl)-methoxy, $C_1$-$C_3$ alkylsulfoxyl, $C_1$-$C_6$ alkylsulfonyl, $C_1$-$C_3$ alkylsulfonyloxy or cyclohexylsulfonyl ; and $R_6$ is halogen.

5. Compounds of the formula I according to claim 1, wherein $R_1$ is $CF_3$, $R_2$ is $NO_2$, $R_3$ is chlorine or bromine, $R_5$ is halogen, $C_1$-$C_4$ alkoxy, chlorine- or bromine-substituted $C_1$-$C_3$ alkyl, methoxy-substituted $C_1$-$C_2$ alkoxy, $C_1$-$C_4$ alkylthio, ($C_2$-$C_3$ alkenyl)-methoxy, ($C_2$-$C_3$ alkynyl)-methoxy or $C_1$-$C_3$ alkylsulfonyl ; and $R_6$ is fluorine, chlorine or bromine.

6. Compounds of the formula I according to claim 1, characterised in that $R_1$ is $NO_2$, $R_2$ is $CF_3$, $R_3$ is halogen and $R_4$ is hydrogen, $R_5$ is $C_1$-$C_3$ alkoxy, $C_1$-$C_3$ alkylthio, $C_1$-$C_3$ alkylsulfoxyl or $C_1$-$C_3$ alkylsulfonyl, and $R_6$ is halogen.

7. Compounds of the formula I according to claim 1, characterised in that $R_1$ is $NO_2$, $R_2$ is $CF_3$, $R_3$ is chlorine and $R_4$ is hydrogen, whilst each or $R_5$ and $R_6$ independently is halogen, $C_1$-$C_6$ alkoxy, chlorine- or bromine-substituted $C_1$-$C_3$ alkyl, $C_1$-$C_3$ alkoxy-substituted $C_1$-$C_3$ alkoxy, $C_1$-$C_6$ alkylthio, cyclopentyloxy, cyclohexyloxy, cyclopentylthio, cyclohexylthio, ($C_2$-$C_5$ alkenyl)-methoxy, ($C_2$ alkenyl)-methylthio, ($C_2$-$C_5$ alkynyl)-methoxy, $C_1$-$C_3$ alkylsulfoxyl, $C_1$-$C_6$ alkylsulfonyl, $C_1$-$C_3$ alkylsulfonyloxy or cyclohexylsulfonyl.

8. Compounds of the formula I according to claim 1, characterised in that $R_1$ is $CF_3$, $R_2$ is $NO_2$, $R_3$ is hydrogen and $R_4$ is hydrogen, and each of $R_5$ and $R_6$ independently is $C_1$-$C_3$ alkoxy, $C_1$-$C_3$ alkylthio, ($C_2$ alkenyl)-methoxy, $C_1$-$C_3$ alkenylsulfoxyl or $C_1$-$C_3$ alkenylsulfonyl.

9. Compounds of the formula I according to claim 1, characterised in that $R_1$ is $CF_3$, or $NO_2$ ; $R_2$ is $CF_3$ or $NO_2$ ; $R_3$ is hydrogen or chlorine ; $R_4$ is the C(O)—$R_7$ group, wherein $R_7$ is $C_1$-$C_3$ alkyl, halogen-substituted $C_1$-$C_3$ alkyl or $C_1$-$C_3$ alkoxy-substituted $C_1$-$C_3$ alkyl ; $R_5$ is halogen, $C_1$-$C_3$ alkoxy or $C_1$-$C_3$ alkylthio, and $R_6$ is chlorine.

10. Compounds of the formula I according to claim 1, characterised in that $R_1$ is $NO_2$, $R_2$ is $CF_3$, $R_3$ is hydrogen and $R_4$ is hydrogen, $R_5$ is halogen, $C_1$-$C_6$-alkoxy, chlorine- or bromine-substituted $C_1$-$C_3$ alkyl, $C_1$-$C_3$ alkoxy-substituted $C_1$-$C_3$ alkoxy, $C_1$-$C_6$ alkylthio, cyclopentyloxy, cyclohexyloxy, cyclopentylthio, cyclohexylthio, ($C_2$-$C_5$ alkenyl)-methoxy, ($C_2$-$C_5$ alkenyl)-methylthio, ($C_2$-$C_5$ alkynyl)-methoxy, $C_1$-$C_3$-alkylsulfoxyl, $C_1$-$C_6$ alkylsulfonyl, $C_1$-$C_3$ alkylsulfonyloxy or cyclohexylsulfonyl ; and $R_6$ is halogen.

11. Compounds of the formula I according to claim 1, selected from the group consisting of ;

N-(3′-chloro-2′,6′-dinitro-4′-trifluoromethylphenyl)-4-amino-2,6-dichloropyrimidine ;

N-(2′,4′-dinitro-6′-trifluoromethylphenyl)-4-amino-2,6-dichloropyridine ;

N-(3′-chloro-2′,6′-dinitro-4′-trifluoromethylphenyl)-4-amino-6-chloro-2-methylmercaptopyrimidine ;

N-(2′,6′-dinitro-4′-trifluoromethylphenyl)-4-amino-6-chloro-2-methylmercaptopyridine ;

N-(3′-chloro-2′,6′-dinitro-4′-trifluoromethylphenyl)-4-amino-6-chloro-2-methoxypyrimidine ;
N-(3′-chloro-2′,6′-dinitro-4′-trifluoromethylphenyl)-4-amino-6-chloro-2-ethoxypyrimidine ;
N-(3′-chloro-2′,6′-dinitro-4′-trifluoromethylphenyl)-4-amino-6-chloro-2-ethylmercaptopyrimidine ;
N-(3′-chloro-2′,6′-dinitro-4′-trifluoromethylphenyl)-4-amino-6-chloro-2-sec-butylmercaptopyrimidine ;
N-(2′,6′-dinitro-4′-trifluoromethylphenyl)-4-amino-2,6-dichloropyrimidine ;
N-(2′,4′-dinitro-6′-trifluoromethylphenyl)-4-amino-2-methoxy-6-chloropyrimidine ;
N-(2′,4′-dinitro-6′-trifluoromethylphenyl)-4-amino-2-ethoxy-6-chloropyrimidine.

12. A process for the preparation of compounds of the formula I, characterised in that a compound of the formula II

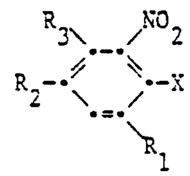

(II)

is reacted with a pyrimidine derivative of the formula III

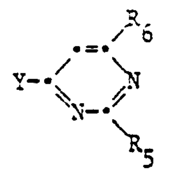

(III)

in the presence of a base, to give a compound of the formula I′

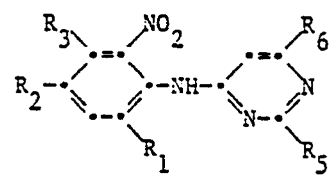

(I′)

and, to prepare N-acylated derivatives, the compound of the formula I′ is N-acylated with a reactive derivative of the carboxylic acid of the formula IV

$$R_7COOH \qquad (IV)$$

in which formulae the substituents $R_1$ to $R_7$ are as defined for formula I and X and Y are $NH_2$ or halogen, with the proviso that, if X is halogen, Y is $NH_2$ and, if X is $NH_2$, Y is halogen.

13. A composition for controlling or preventing attack by micro-organisms, characterised in that it contains as at least one active ingredient a compound of the formula I according to claim 1.

14. A composition according to claim 13, characterised in that it contains as at least one active ingredient a compound of the formula I according to claim 2.

15. A composition for controlling micro-organisms according to claim 13, characterised in that it contains as at least one active ingredient a compound of the formula I according to any one of claims 3 to 11.

16. A composition according to claim 13, characterised in that it contains from 0.1 to 99 % of a compound of the formula I, from 99.9 to 1 % of a solid or liquid adjuvant, and from 0 to 25 % of a surfactant.

17. A composition according to claim 16, characterised in that it contains from 0.1 to 95 % of a compound of the formula I, from 99.8 to 5 % of a solid or liquid adjuvant, and from 0.1 to 25 % of a surfactant.

18. A method of controlling or preventing attack of cultivated plants by phytopathogenic micro-organisms, characterised in that a compound of the formula I as defined in claim 1 is applied to the plants or to the locus thereof.

**Claims** (for the Contracting State AT)

1. A fungicidal composition containing a compound of the general formula I

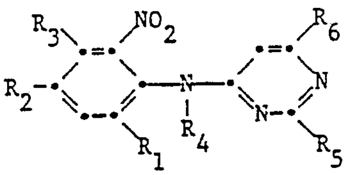

(I)

wherein

$R_1$ is $NO_2$ or $CF_3$ ;

$R_2$ is $NO_2$ or $CF_3$ ;

$R_3$ is hydrogen or halogen ;

$R_4$ is hydrogen or the —C(O)$R_7$ group, wherein $R_7$ is $C_1$-$C_4$ alkyl which is unsubstituted or substituted by halogen, $C_1$-$C_3$ alkoxy or by $C_1$-$C_3$ alkylthio ;

$R_5$ and $R_6$ are each independently halogen, $C_1$-$C_6$ alkoxy, $C_1$-$C_6$ alkyl which is substituted by halogen or by $C_1$-$C_3$ alkoxy, $C_1$-$C_6$ alkoxy which is substituted by halogen or by $C_1$-$C_3$ alkoxy, $C_1$-$C_6$ alkylthio, $C_1$-$C_6$ alkylthio which is substituted by halogen or by $C_1$-$C_3$ alkoxy, $C_3$-$C_6$ cycloalkoxy, $C_3$-$C_6$ cycloalkylthio, ($C_2$-$C_5$ alkenyl)-methoxy, ($C_2$-$C_5$ alkenyl)-methylthio, ($C_2$-$C_5$ alkynyl)-methoxy, $C_3$-$C_6$ alkynyloxy, $C_1$-$C_6$ alkylsulfoxyl, $C_1$-$C_6$ alkylsulfonyl, $C_1$-$C_6$ alkylsulfonyloxy, $C_3$-$C_6$ alkenylsulfoxyl, $C_3$-$C_6$ alkenylsulfonyl, $C_3$-$C_6$ cycloalkylsulfoxyl or $C_3$-$C_6$ cycloalkylsulfonyl, together with customary carriers and adjuncts.

2. A composition according to claim 1, containing a compound of the formula I, characterised in that $R_1$ is $NO_2$, $R_2$ is $CF_3$, $R_3$ is chlorine and $R_4$ is hydrogen, $R_5$ is halogen, $C_1$-$C_6$ alkoxy, chlorine- or bromine-substituted $C_1$-$C_3$ alkyl, $C_1$-$C_3$ alkoxy-substituted $C_1$-$C_3$ alkoxy, $C_1$-$C_6$ alkylthio, cyclopentyloxy, cyclohexyloxy, cyclopentylthio, cyclohexylthio, ($C_2$-$C_5$ alkenyl)-methoxy, ($C_2$-$C_5$ alkenyl)-methylthio, ($C_2$-$C_5$ alkynyl)-methoxy, $C_1$-$C_3$ alkylsulfoxyl, $C_1$-$C_6$ alkylsulfonyl, $C_1$-$C_3$ alkylsulfonyloxy or cyclohexylsulfonyl ; and $R_6$ is halogen.

3. A composition according to claim 1, containing a compound of the formula I, wherein $R_1$ is $NO_2$, $R_2$ is $CF_3$, $R_3$ is chlorine or bromine, $R_5$ is halogen, $C_1$-$C_4$ alkoxy, chlorine- or bromine-substituted $C_1$-$C_3$ alkyl, methoxy-substituted $C_1$-$C_2$ alkoxy, $C_1$-$C_4$ alkylthio, ($C_2$-$C_3$ alkenyl)-methoxy, ($C_2$-$C_3$ alkynyl)-methoxy or $C_1$-$C_3$ alkylsulfonyl ; and $R_6$ is fluorine, chlorine or bromine.

4. A composition according to claim 1, containing a compound of the formula I, characterised in that $R_1$ is $CF_3$, $R_2$ is $NO_2$, $R_3$ is hydrogen and $R_4$ is hydrogen, $R_5$ is halogen, $C_1$-$C_6$ alkoxy, chlorine- or bromine-substituted $C_1$-$C_3$ alkyl, $C_1$-$C_3$ alkoxy-substituted $C_1$-$C_3$ alkoxy, $C_1$-$C_6$ alkylthio, cyclopentyloxy, cyclohexyloxy, cyclopentylthio, cyclohexylthio, ($C_2$-$C_5$ alkenyl)-methoxy, ($C_2$-$C_5$ alkenyl)-methylthio, ($C_2$-$C_5$ alkynyl)-methoxy, $C_1$-$C_3$ alkylsulfoxyl, $C_1$-$C_6$ alkylsulfonyl, $C_1$-$C_3$ alkylsulfonyloxy or cyclohexylsulfonyl ; and $R_6$ is halogen.

5. A composition according to claim 1, containing a compound of the formula I, wherein $R_1$ is $CF_3$, $R_2$ is $NO_2$, $R_3$ is chlorine or bromine, $R_5$ is halogen, $C_1$-$C_4$ alkoxy, chlorine- or bromine-substituted $C_1$-$C_3$ alkyl, methoxy-substituted $C_1$-$C_2$ alkoxy, $C_1$-$C_4$ alkylthio, ($C_2$-$C_3$ alkenyl)-methoxy, ($C_2$-$C_3$ alkynyl)-methoxy or $C_1$-$C_3$ alkylsulfonyl ; and $R_6$ is fluorine, chlorine or bromine.

6. A composition according to claim 1, containing a compound of the formula I, characterised in that $R_1$ is $NO_2$, $R_2$ is $CF_3$, $R_3$ is halogen and $R_4$ is hydrogen, $R_5$ is $C_1$-$C_3$ alkoxy, $C_1$-$C_3$ alkylthio, $C_1$-$C_3$ alkylsulfoxyl or $C_1$-$C_3$ alkylsulfonyl, and $R_6$ is halogen.

7. A composition according to claim 1, containing a compound of the formula I, characterised in that $R_1$ is $NO_2$, $R_2$ is $CF_3$, $R_3$ is chlorine and $R_4$ is hydrogen, whilst each of $R_5$ and $R_6$ independently is halogen, $C_1$-$C_6$ alkoxy, chlorine- or bromine-substituted $C_1$-$C_3$ alkyl, $C_1$-$C_3$ alkoxy-substituted $C_1$-$C_3$ alkoxy, $C_1$-$C_6$ alkylthio, cyclopentyloxy, cyclohexyloxy, cyclopentylthio, cyclohexylthio, ($C_2$-$C_5$ alkenyl)-methoxy, ($C_2$ alkenyl)-methylthio, ($C_2$-$C_5$ alkynyl)-methoxy, $C_1$-$C_3$ alkylsulfoxyl, $C_1$-$C_6$ alkylsulfonyl, $C_1$-$C_3$ alkylsulfonyloxy or cyclohexylsulfonyl.

8. A composition according to claim 1, containing a compound of the formula I, characterised in that $R_1$ is $CF_3$, $R_2$ is $NO_2$, $R_3$ is hydrogen and $R_4$ is hydrogen, and each of $R_5$ and $R_6$ independently is $C_1$-$C_3$ alkoxy, $C_1$-$C_3$ alkylthio, ($C_2$ alkenyl)-methoxy, $C_1$-$C_3$ alkenylsulfoxyl or $C_1$-$C_3$ alkenylsulfonyl.

9. A composition according to claim 1, containing a compound of the formula I, characterised in that $R_1$ is $CF_3$ or $NO_2$ ; $R_2$ is $CF_3$ or $NO_2$ ; $R_3$ is hydrogen or chlorine ; $R_4$ is the C(O)—$R_7$ group, wherein $R_7$ is $C_1$-$C_3$ alkyl, halogen-substituted $C_1$-$C_3$ alkyl or $C_1$-$C_3$ alkoxy-substituted $C_1$-$C_3$ alkyl ; $R_5$ is halogen, $C_1$-$C_3$ alkoxy or $C_1$-$C_3$ alkylthio, and $R_6$ is chlorine.

10. A composition according to claim 1, containing a compound of the formula I, characterised in that $R_1$ is $NO_2$, $R_2$ is $CF_3$, $R_3$ is hydrogen and $R_4$ is hydrogen, $R_5$ is halogen, $C_1$-$C_6$ alkoxy, chlorine- or bromine-substituted $C_1$-$C_3$ alkyl, $C_1$-$C_3$ alkoxy-substituted $C_1$-$C_3$ alkoxy, $C_1$-$C_6$ alkylthio, cyclopentyloxy, cyclohexyloxy, cyclopentylthio, cyclohexylthio, ($C_2$-$C_5$ alkenyl)-methoxy, ($C_2$-$C_5$ alkenyl)-methylthio, ($C_2$-$C_5$ alkynyl)-methoxy, $C_1$-$C_3$ alkylsulfoxyl, $C_1$-$C_6$ alkylsulfonyl, $C_1$-$C_3$ alkylsulfonyloxy or cyclohexylsulfonyl ; and $R_6$ is halogen.

11. A composition according to claim 1, containing a compound of the formula I, selected from the group consisting of :

N-(3'-chloro-2',6'-dinitro-4'-trifluoromethylphenyl)-4-amino-2,6-dichloropyrimidine ;

N-(2',4'-dinitro-6'-trifluoromethylphenyl)-4-amino-2,6-dichloropyridine ;

N-(3'-chloro-2',6'-dinitro-4'-trifluoromethylphenyl)-4-amino-6-chloro-2-methylmercaptopyrimidine ;
N-(2',6'-dinitro-4'-trifluoromethylphenyl)-4-amino-6-chloro-2-methylmercaptopyridine ;
N-(3'-chloro-2',6'-dinitro-4'-trifluoromethylphenyl)-4-amino-6-chloro-2-methoxypyrimidine ;
N-(3'-chloro-2',6'-dinitro-4'-trifluoromethylphenyl)-4-amino-6-chloro-2-ethoxypyrimidine ;
N-(3'-chloro-2',6'-dinitro-4'-trifluoromethylphenyl)-4-amino-6-chloro-2-ethylmercaptopyrimidine ;
N-(3'-chloro-2',6'-dinitro-4'-trifluoromethylphenyl)-4-amino-6-chloro-2-sec-butylmercaptopyrimidine ;
N-(2',6'-dinitro-4'-trifluoromethylphenyl)-4-amino-2,6-dichloropyrimidine ;
N-(2',4'-dinitro-6'-trifluoromethylphenyl)-4-amino-2-methoxy-6-chloropyrimidine ;
N-(2',4'-dinitro-6'-trifluoromethylphenyl)-4-amino-2-ethoxy-6-chloropyrimidine.

12. A process for the preparation of compounds of the formula I, characterised in that a compound of the formula II

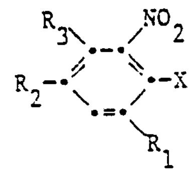

(II)

is reacted with a pyrimidine derivative of the formula III

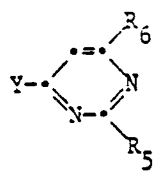

(III)

in the presence of a base, to give a compound of the formula I'

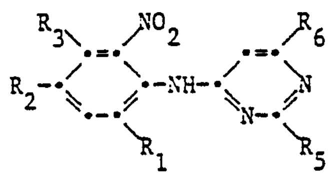

(I')

and, to prepare N-acylated derivatives, the compound of the formula I' is N-acylated with a reactive derivative of the carboxylic acid of the formula IV

$$R_7COOH \quad (IV)$$

in which formulae the substituents $R_1$ to $R_7$ are as defined for formula I and X and Y are $NH_2$ or halogen, with the proviso that, if X is halogen, Y is $NH_2$ and, if X is $NH_2$, Y is halogen.

13. A method of controlling or preventing attack of cultivated plants by phytopathogenic micro-organisms, characterised in that a compound of the formula I as defined in claim 1 is applied to the plants or to the locus thereof.

**Revendications** (pour les Etats contractants : BE, CH, LI, DE, FR, IT, LU, NL, SE)

1. Composés de formule générale I

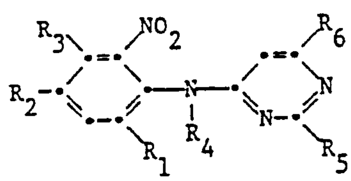

(I)

où

R1 représente $NO_2$ ou $CF_3$ ;
R2 représente $NO_2$ ou $CF_3$ ;
R3 représente un hydrogène ou un halogène ;
R4 représente un hydrogène ou le groupe —C(O)R7, où R7 représente un alcoyle en C1 à C4 non

substitué ou substitué par un halogène, alcoxy en C1 à C3 ou alcoylthio en C1 à C3 ;

R5 et R6 représentent indépendamment l'un de l'autre un halogène, alcoxy en C1 à C6, alcoyle en C1 à C6 substitué par un halogène ou un alcoxy en C1 à C3, un alcoxy en C1 à C6 substitué par un halogène ou un alcoxy en C1 à C3, un alcoylthio en C1 à C6, un alcoylthio en C1 à C6 substitué par un halogène ou un alcoxy en C1 à C3, un cycloalcoxy en C3 à C6, cycloalcoylthio en C3 à C6, (alcényle en C2 à C5)-méthoxy, (alcényle en C2 à C5)-méthylthio, (alcynyle en C2 à C5)-méthoxy, alcoylsulfoxyle en C1 à C6, alcoylsulfonyle en C1 à C6, sulfonyloxy en C1 à C6, alcénylsulfoxyle en C3 à C6, alcénylsulfonyle en C3 à C6, cycloalcoylsulfoxyle en C3 à C6 ou cycloalcoylsulfonyle en C3 à C6.

2. Composés de formule I selon la revendication 1, caractérisés en ce que R1 représente $NO_2$, R2 représente $CF_3$, R3 représente un chlore et R4 représente un hydrogène, R5 représente un halogène, alcoxy en C1 à C6, alcoyle en C1 à C3 substitué par un chlore ou un brome, alcoxy en C1 à C3 substitué par un alcoxy en C1 à C3, alcoylthio en C1 à C6, cyclopentyloxy, cyclohexyloxy, cyclopentylthio, cyclohexylthio, (alcényle en C2 à C5)-méthoxy, (alcényle en C2 à C5)-méthylthio, (alcynyle en C2 à C5)-méthoxy, alcoylsulfoxyle en C1 à C3, alcoylsulfonyle en C1 à C6, alcoylsulfonyloxy en C1 à C3 ou cyclohexylsulfonyle ; et R6 représente un halogène.

3. Composés de formule I selon la revendication 1, où R1 représente $NO_2$, R2 représente $CF_3$, R3 représente un chlore ou un brome, R5 représente un halogène, un alcoxy en C1 à C4, un alcoyle en C1 à C3 substitué par un chlore ou un brome, un alcoxy en C1 à C2 substitué par un méthoxy, un alcoylthio en C1 à C4, (alcényle en C2 à C3)-méthoxy, (alcynyle en C2 à C3)-méthoxy ou alcoylsulfonyle en C1 à C3 ; et R6 représente un fluor, chlore ou brome.

4. Composés de formule I selon la revendication 1, caractérisés en ce que R1 représente $CF_3$, R2 représente $NO_2$, R3 représente un hydrogène et R4 un hydrogène, R5 représente un halogène, un alcoxy en C1 à C6, un alcoyle en C1 à C3 substitué par un chlore ou un brome, un alcoxy en C1 à C3 substitué par un alcoxy en C1 à C3, un alcoylthio en C1 à C6, un cyclopentyloxy, cyclohexyloxy, cyclopentylthio, cyclohexylthio, (alcényle en C2 à C5)-méthoxy, (alcényle en C2 à C5)-méthylthio, (alcynyle en C2 à C5)-méthoxy, alcoylsulfoxyle en C1 à C3, alcoylsulfonyle en C1 à C6, alcoylsulfonyloxy en C1 à C3 ou cyclohexylsulfonyle ; et R6 représente un halogène.

5. Composés de formule I selon la revendication 1, où R1 représente $CF_3$, R2 représente $NO_2$, R3 représente un chlore ou un brome, R5 un halogène, alcoxy en C1 à C4, alcoyle en C1 à C3 substitué par un chlore ou un brome, alcoxy en C1 à C2 substitué par un méthoxy, alcoylthio en C1 à C4, (alcényle en C2 à C3)-méthoxy, (alcynyle en C2 à C3)-méthoxy ou alcoylsulfonyle en C1 à C3 ; et R6 représente un fluor, chlore ou brome.

6. Composés de formule I selon la revendication 1, caractérisés en ce que R1 représente $NO_2$, R2 représente $CF_3$, R3 représente un halogène et R4 un hydrogène, R5 un alcoxy en C1 à C3, alcoylthio en C1 à C3, alcoylsulfoxyle en C1 à C3 ou alcoylsulfonyle en C1 à C3 et R6 représente un halogène.

7. Composés de formule I selon la revendication 1, caractérisés en ce que R1 représente $NO_2$, R2 représente $CF_3$, R3 représente un chlore et R4 un hydrogène, tandis que R5 et R6 représentent indépendamment l'un de l'autre un halogène, alcoxy en C1 à C6, alcoyle en C1 à C3 substitué par un chlore ou un brome, alcoxy en C1 à C3 substitué par un alcoxy en C1 à C3, alcoylthio en C1 à C6, cyclopentyloxy, cyclohexyloxy, cyclopentylthio, cyclohexylthio, (alcényle en C2 à C5)-méthoxy, (alcényle en C2)-méthylthio, (alcynyle en C2 à C5)-méthoxy, alcoylsulfoxyle en C1 à C3, alcoylsulfonyle en C1 à C6, alcoylsulfonyloxy en C1 à C3 ou cyclohexylsulfonyle.

8. Composés de formule I selon la revendication 1, caractérisés en ce que R1 représente $CF_3$, R2 représente $NO_2$, R3 représente un hydrogène et R4 un hydrogène et R5 et R6 représentent indépendamment l'un de l'autre un alcoxy en C1 à C3, alcoylthio en C1 à C3, alcényloxy en C3, alcoxy en C1 à C3, alcoylthio en C1 à C3, (alcényle en C2)-méthoxy, alcénylsulfoxyle en C1 à C3 ou alcénylsulfonyle en C1 à C3.

9. Composés de formule I selon la revendication 1, caractérisés en ce que R1 représente $CF_3$ ou $NO_2$ ; R2 représente $CF_3$ ou $NO_2$ ; R3 représente un hydrogène ou un chlore ; R4 représente le groupe C(O)—R7, où R7 représente un alcoyle en C1 à C3, un alcoyle en C1 à C3 substitué par un halogène ou un alcoyle en C1 à C3 substitué par un alcoxy en C1 à C3 ; R5 représente un halogène, un alcoxy en C1 à C3 ou un alcoylthio en C1 à C3 et R6 représente un chlore.

10. Composés de formule I selon la revendication 1, caractérisés en ce que R1 représente $NO_2$, R2 représente $CF_3$, R3 représente un hydrogène et R4 un hydrogène, R5 représente un halogène, un alcoxy en C1 à C6, un alcoyle en C1 à C3 substitué par un chlore ou un brome, un alcoxy en C1 à C3 substitué par un alcoxy en C1 à C3, un alcoylthio en C1 à C6, un cyclopentyloxy, cyclohexyloxy, cyclopentylthio, cyclohexylthio, (alcényle en C2 à C5)-méthoxy, (alcényle en C2 à C5)-méthylthio, (alcynyle en C2 à C5)-méthoxy, alcoylsulfoxyle en C1 à C3, alcoylsulfonyle en C1 à C6, alcoylsulfonyloxy en C1 à C3 ou cyclohexylsulfonyle ; et R6 représente un halogène.

11. Composés de formule I selon la revendication 1, choisis dans la série :

N-(3'-chloro-2',6'-dinitro-4'-trifluorométhylphényl)-4-amino-2,6-di-chloro-pyrimidine ;

N-(2',4'-dinitro-6'-trifluorométhylphényl)-4-amino-2,6-dichloro-pyrimidine ;

N-(3'-chloro-2',6'-dinitro-4'-trifluorométhylphényl)-4-amino-6-chloro-2-méthylmercapto-pyrimidine ;

N-(2',6'-dinitro-4'-trifluorométhylphényl)-4-amino-6-chloro-2-méthyl-mercapto-pyrimidine ;

N-(3'-chloro-2',6'-dinitro-4'-trifluorométhylphényl)-4-amino-6-chloro-2-méthoxy-pyrimidine ;

N-(3′-chloro-2′,6′-dinitro-4′-trifluorométhylphényl)-4-amino-6-chloro-2-éthoxy-pyrimidine ;
N-(3′-chloro-2′,6′-dinitro-4′-trifluorométhylphényl)-4-amino-6-chloro-2-éthylmercapto-pyrimidine ;
N-(3′-chloro-2′-6′-dinitro-4′-trifluorométhylphényl)-4-amino-6-chloro-2-sec.butylmercapto-pyrimidine ;
N-(2′,6′-dinitro-4′-trifluorométhylphényl)-4-amino-2,6-dichloro-pyrimidine ;
N-(2′,4′-dinitro-6′-trifluorométhylphényl)-4-amino-2-méthoxy-6-chloropyrimidine ;
N-(2′,4′-dinitro-6′-trifluorméthylphényl)-4-amino-2-éthoxy-6-chloropyrimidine.

12. Procédé de préparation de composés de formule I, caractérisé en ce qu'on fait réagir un composé de formule II

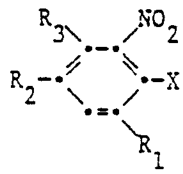

(II)

en présence d'une base avec un dérivé de pyrimidine de formule III

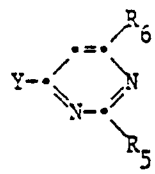

(III)

pour donner un composé de formule I′

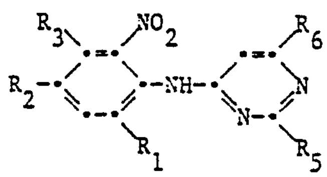

(I′)

et en ce qu'on N-acyle ce dernier pour préparer des dérivés N-acylés avec un dérivé réactif de l'acide carboxylique IV

$$R_7COOH \quad (IV)$$

les substituants R1 à R7 ayant les significations données sous la formule I et X et Y représentant $NH_2$ ou un halogène, où au cas où X représente un halogène, Y représente $NH_2$ et au cas où X représente $NH_2$, Y représente un halogène.

13. Agent de lutte ou de prévention d'une attaque par les microorganismes, caractérisé en ce qu'il contient comme composant actif au moins un composé de formule I selon la revendication 1.

14. Agent selon la revendication 13, caractérisé en ce qu'il contient comme composant actif au moins un composé de formule I selon la revendication 2.

15. Agent de lutte contre les microorganismes selon la revendication 13, caractérisé en ce qu'il contient comme composant actif au moins un composé de formule I selon l'une des revendications 3 à 11.

16. Agent selon la revendication 13, caractérisé en ce qu'il contient de 0,1 à 99 % d'une matière active de formule I, 99,9 à 1 % d'un additif solide ou liquide et de 0 à 25 % d'un agent tensio-actif.

17. Agent selon la revendication 16, caractérisé en ce qu'il contient de 0,1 à 95 % d'une matière active de formule I, de 99,8 à 5 % d'un additif solide ou liquide et de 0,1 à 25 % d'un agent tensio-actif.

18. Procédé de lutte ou de prévention d'une attaque des plantes cultivées par des microorganismes phytopathogènes, caractérisé en ce qu'on applique un composé de formule I défini selon la revendication 1 sur la plante ou l'endroit où elle se trouve.

**Revendications** (pour l'Etat contractant AT)

1. Agent fongicide contenant outre les supports et additifs habituels un composé de formule générale I

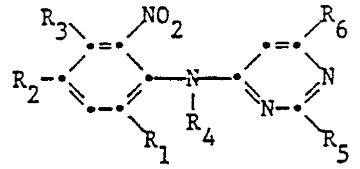

(I)

où

R1 représente $NO_2$ ou $CF_3$ ;

R2. représente $NO_2$ ou $CF_3$ ;

R3 représente un hydrogène ou un halogène ;

R4 représente un hydrogène ou le groupe —C(O)R7, où R7 représente un alcoyle en C1 à C4 non substitué ou substitué par un halogène, alcoxy en C1 à C3 ou alcoylthio en C1 à C3 ;

R5 et R6 représentent indépendamment l'un de l'autre un halogène, alcoxy en C1 à C6, alcoyle en C1 à C6 substitué par un halogène ou un alcoxy en C1 à C3, un alcoxy en C1 à C6 substitué par un halogène ou un alcoxy en C1 à C3, un alcoylthio en C1 à C6, un alcoylthio en C1 à C6 substitué par un halogène ou un alcoxy en C1 à C3, un cycloalcoxy en C3 à C6, cycloalcoylthio en C3 à C6, (alcényle en C2 à C5)-méthoxy, (alcényle en C2 à C5)-méthylthio, (alcynyle en C2 à C5)-méthoxy, alcoylsulfoxyde en C1 à C6, alcoylsulfonyle en C1 à C6, sulfonyloxy en C1 à C6, alcénylsulfoxyle en C3 à C6, alcénylsulfonyle en C3 à C6, cycloalcoylsulfoxyle en C3 à C6 ou cycloalcoylsulfonyle en C3 à C6.

2. Agent selon la revendication 1, contenant un composé de formule I, caractérisé en ce que R1 représente $NO_2$, R2 représente $CF_3$, R3 représente un chlore et R4 représente un hydrogène, R5 représente un halogène, alcoxy en C1 à C6, alcoyle en C1 à C3 substitué par un chlore ou un brome, alcoxy en C1 à C3 substitué par un alcoxy en C1 à C3, alcoylthio en C1 à C6, cyclopentyloxy, cyclohexyloxy, cyclopentylthio, cyclohexylthio, (alcényle en C2 à C5)-méthoxy, (alcényle en C2 à C5)-méthylthio, (alcynyle en C2 à C5)-méthoxy, alcoylsulfoxyle en C1 à C3, alcoylsulfonyle en C1 à C6, alcoylsulfonyloxy en C1 à C3 ou cyclohexylsulfonyle ; et R6 représente un halogène.

3. Agent selon la revendication 1, contenant un composé de formule I, où R1 représente $NO_2$, R2 représente $CF_3$, R3 représente un chlore ou un brome, R5 représente un halogène, un alcoxy en C1 à C4, un alcoyle en C1 à C2 substitué par un chlore ou un brome, un alcoxy en C1 à C2 substitué par un méthoxy, un alcoylthio en C1 à C4, (alcényle en C2 à C3)-méthoxy, (alcynyle en C2 à C3)-méthoxy ou alcoylsulfonyle en C1 à C3 ; et R6 représente un fluor, chlore ou brome.

4. Agent selon la revendication 1, contenant un composé de formule I, caractérisé en ce que R1 représente $CF_3$, R2 représente $NO_2$, R3 représente un hydrogène et R4 un hydrogène, R5 représente un halogène, un alcoxy en C1 à C6, un alcoyle en C1 à C3 substitué par un chlore ou un brome, un alcoxy en C1 à C3 substitué par un alcoxy en C1 à C3, un alcoylthio en C1 à C6, un cyclopentyloxy, cyclohexyloxy, cyclopentylthio, cyclohexylthio, (alcényle en C2 à C5)-méthoxy, (alcényle en C2 à C5)-méthylthio, (alcynyle en C2 à C5)-méthoxy, alcoylsulfoxyle en C1 à C3, alcoylsulfonyle en C1 à C6, alcoylsulfonyloxy en C1 à C3 ou cyclohexylsulfonyle ; et R6 représente un halogène.

5. Agent selon la revendication 1, contenant un composé de formule I, où R1 représente $CF_3$, R2 représente $NO_2$, R3 représente un chlore ou un brome, R5 un halogène, alcoxy en C1 à C4, alcoyle en C1 à C3 substitué par un chlore ou un brome, alcoxy en C1 à C2 substitué par un méthoxy, alcoylthio en C1 à C4, (alcényle en C2 à C3)-méthoxy, (alcynyle en C2 à C3)-méthoxy ou alcoylsulfonyle en C1 à C3 ; et R6 représente un fluor, chlore ou brome.

6. Agent selon la revendication 1, contenant un composé de formule I, caractérisé en ce que R1 représente $NO_2$, R2 représente $CF_3$, R3 représente un halogène et R4 représente un hydrogène, R5 représente un alcoxy en C1 à C3, alcoylthio en C1 à C3, alcoylsulfoxyle en C1 à C3 ou alcoylsulfonyle en C1 à C3 et R6 représente un halogène.

7. Agent selon la revendication 1, contenant un composé de formule I, caractérisé en ce que R1 représente $NO_2$, R2 représente $CF_3$, R3 représente un chlore et R4 un hydrogène, tandis que R5 et R6 représentent indépendamment l'un de l'autre un halogène, alcoxy en C1 à C6, alcoyle en C1 à C3 substitué par un chlore ou un brome, alcoxy en C1 à C3 substitué par un alcoxy en C1 à C3, alcoylthio en C1 à C6, cyclopentyloxy, cyclohexyloxy, cyclopentylthio, cyclohexylthio, (alcényle en C2 à C5)-méthoxy, (alcényle en C2)-méthylthio, (alcynyle en C2 à C5)-méthoxy, alcoylsulfoxyle en C1 à C3, alcoylsulfonyle en C1 à C6, alcoylsulfonyloxy en C1 à C3 ou cyclohexylsulfonyle.

8. Agent selon la revendication 1, contenant un composé de formule I, caractérisé en ce que R1 représente $CF_3$, R2 représente $NO_2$, R3 représente un hydrogène et R4 un hydrogène et R5 et R6 représentent indépendamment l'un de l'autre un alcoxy en C1 à C3, alcoylthio en C1 à C3, alcényloxy en C3, alcoxy en C1 à C3, alcoylthio en C1 à C3, (alcényle en C2)-méthoxy, alcénylsulfoxyle en C1 à C3 ou alcénylsulfonyle en C1 à C3.

9. Agent selon la revendication 1, contenant un composé de formule I, caractérisé en ce que R1 représente $CF_3$ ou $NO_2$ ; R2 représente $CF_3$ ou $NO_2$ ; R3 représente un hydrogène ou un chlore, R4 représente le groupe C(O)—R7, où R7 représente un alcoyle en C1 à C3, un alcoyl en C1 à C3 substitué par un halogène ou un alcoyle en C1 à C3 substitué par un alcoxy en C1 à C3 ; R5 représente un halogène, un alcoxy en C1 à C3 ou un alcoylthio en C1 à C3 et R6 représente un chlore.

10. Agent selon la revendication 1, contenant un composé de formule I, caractérisé en ce que R1 représente $NO_2$, R2 représente $CF_3$, R3 représente un hydrogène et R4 un hydrogène, R5 représente un halogène, un alcoxy en C1 à C6, un alcoyle en C1 à C3 substitué par un chlore ou un brome, un alcoxy en C1 à C6, un cyclopentyloxy, cyclohexyloxy, cyclopentylthio, cyclohexylthio, (alcényle en C2 à C5)-méthoxy, (alcényle en C2 à C5)-méthylthio, (alcynyle en C2 à C5)-méthoxy, alcoylsulfoxyle en C1 à C3, alcoylsulfonyle en C1 à C6, alcoylsulfonyloxy en C1 à C3 ou cyclohexylsulfonyle ; et R6 représente un halogène.

11. Agent selon la revendication 1, contenant un composé de formule I, choisi dans la série :
N-(3'-chloro-2',6'-dinitro-4'-trifluorométhylphényl)-4-amino-2,6-di-chloro-pyrimidine ;
N-(2',4'-dinitro-6'-trifluorométhylphényl)-4-amino-2,6-dichloro-pyrimidine ;
N-(3'-chloro-2',6'-dinitro-4'-trifluorométhylphényl)-4-amino-6-chloro-2-méthylmercapto-pyrimidine ;
N-(2',6'-dinitro-4'-trifluorométhylphényl)-4-amino-6-chloro-2-méthyl-mercapto-pyrimidine ;
N-(3'-chloro-2',6'-dinitro-4'-trifluorométhylphényl)-4-amino-6-chloro-2-méthoxy-pyrimidine ;
N-(3'-chloro-2',6'-dinitro-4'-trifluorométhylphényl)-4-amino-6-chloro-2-éthoxy-pyrimidine ;
N-(3'-chloro-2',6'-dinitro-4'-trifluorométhylphényl)-4-amino-6-chloro-2-éthylmercapto-pyrimidine ;
N-(3'-chloro-2',6'-dinitro-4'-trifluorométhylphényl)-4-amino-6-chloro-2-sec.butylmercapto-pyrimidine ;
N-(2',6'-dinitro-4'-trifluorométhylphényl)-4-amino-2,6-dichloro-pyrimidine ;
N-(2',4'-dinitro-6'-trifluorométhylphényl)-4-amino-2-méthoxy-6-chloro-pyrimidine ;
N-(2',4'-dinitro-6'-trifluorométhylphényl)-4-amino-2-éthoxy-6-chloro-pyrimidine.

12. Procédé de préparation de composés de formule I, caractérisé en ce qu'on fait réagir un composé de formule II

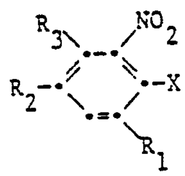

(II)

en présence d'une base avec un dérivé de pyrimidine de formule III

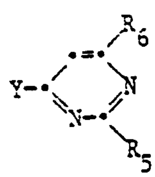

(III)

pour donner un composé de formule I'

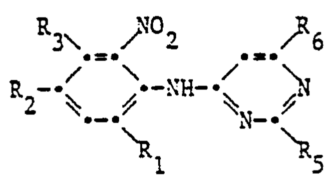

(I')

et en ce qu'on N-acyle ce dernier pour préparer des dérivés N-acylés avec un dérivé réactif de l'acide carboxylique IV

$$R_7COOH \qquad (IV)$$

les substituants R1 à R7 ayant les significations données sous la formule I et X et Y représentant $NH_2$ ou un halogène, où au cas où X représente un halogène, Y représente $NH_2$ et au cas où X représente $NH_2$, Y représente un halogène.

13. Procédé de lutte ou de prévention d'une attaque de plantes cultivées par les microorganismes phytopathogènes, caractérisé en ce qu'on applique un composé de formule I défini selon la revendication 1 sur la plante ou l'endroit où elle se trouve.